# EUROPEAN PATENT SPECIFICATION

(11) **EP 2 724 162 B1**
(45) Date of publication and mention of the grant of the patent: **06.07.2016**
(21) Application number: 12733199.9
(22) Date of filing: 27.06.2012
(51) Int. Cl.: G01N 33/68

(54) **DIAGNOSTIC METHOD FOR DOWN'S SYNDROME**
DIAGNOSEVERFAHREN FÜR DOWN-SYNDROM
MÉTHODE DIAGNOSTIQUE POUR LE SYNDROME DE DOWN

(30) Priority: 27.06.2011 US 201161501301 P
(43) Date of publication of application: 30.04.2014
(73) Proprietor: UCL Business PLC, London, W1T 4TP (GB)
(72) Inventor: CHITTY, Lyn, Rickmansworth Herts WD3 9XR (GB); HEYWOOD, Wendy, Crawley West Sussex RH10 7ED (GB); MILLS, Kevin, Little Chalfont Bucks HP6 6SL (GB)
(74) Representative: Bentham, Andrew
(86) International application number: PCT/GB2012/051501
(87) International publication number: WO 2013/001293

(56) References cited:
- WO-A2-2007/112082
- CHIU R W K ET AL: "Non-invasive prenatal diagnosis by single molecule counting technologies", TRENDS IN GENETICS, ELSEVIER SCIENCE PUBLISHERS B.V. AMSTERDAM, NL, vol. 25, no. 7, 1 July 2009 (2009-07-01), pages 324-331, XP026301457, ISSN: 0168-9525, DOI: 10.1016/J.TIG.2009.05.004 [retrieved on 2009-06-18]
- S. S.C. CHIM ET AL: "Systematic Search for Placental DNA-Methylation Markers on Chromosome 21: Toward a Maternal Plasma-Based Epigenetic Test for Fetal Trisomy 21", CLINICAL CHEMISTRY, vol. 54, no. 3, 1 March 2008 (2008-03-01), pages 500-511, XP055003626, ISSN: 0009-9147, DOI: 10.1373/clinchem.2007.098731
- GRIFFITHS WILLIAM J ET AL: "Mass spectrometry: from proteomics to metabolomics and lipidomics", CHEMICAL SOCIETY REVIEWS, vol. 38, no. 7, 2009, pages 1882-1896, XP002683308, ISSN: 0306-0012
- HEYWOOD WENDY ET AL: "The development of a peptide SRM-based tandem mass spectrometry assay for prenatal screening of Down syndrome", JOURNAL OF PROTEOMICS, vol. 75, no. 11, June 2012 (2012-06), pages 3248-3257 URL, XP002683309,
- HEYWOOD WENDY ET AL: "Identification of new biomarkers for Down's syndrome in maternal plasma", JOURNAL OF PROTEOMICS, vol. 75, no. 9, May 2012 (2012-05), pages 2621-2628 URL, XP002683310,

## Description

### Field of the Invention

This invention relates to Down's syndrome screening (DSS). In particular, the invention relates to a new biomarker for Down's syndrome (DS) that may be detected during either the first or second trimester. The present invention also relates to a method for diagnosing DS.

### Background of the Invention

Down's syndrome is the most common chromosomal abnormality compatible with long-term survival. There are 100,000 live births per annum in the North Thames region of England alone and 706,000 live births in England and Wales. The UK National Screening Committee (NSC) protocol dictates that all pregnant women are offered screening for Down's syndrome, ideally at 11-13 weeks gestation.

In the UK, the current standard requires that any test offered has a 75% detection rate for no more than a 3% false positive rate. Guidelines state that the objective is to offer a test that has a 90% detection rate for a 2% false positive rate.

Current UK policy is to deliver a test using an algorithm combining maternal age, two maternal serum markers and one ultrasound marker, nuchal translucency (NT), which gives a detection rate of around 85% for a 3% false positive rate (http://fetalanomaly.screening.nhs.uk/standardsandpolicies). The current tests can be performed in the late first, or second trimester of pregnancy or a combination of both (Macrae et al (2010) Prenat. Diagn. 30(5): 459-466).

Chiu *et al* discloses non-invasive prenatal diagnosis by single molecule counting technologies. Chim *et al* discloses a Systematic search for placental DNA-methylation markers on Chromosome 21. Griffiths *et al* discloses mass spectrometry techniques. WO2007/112082 discloses methods and compositions for the identification of cancer markers.

### Summary of the Invention

The present inventors have shown that the level or concentration of plasma protease C1 inhibitor (C1 inhibitor) is significantly raised in pregnant females carrying DS fetuses compared to the level or concentration in pregnant females carrying non-DS fetuses. This observation can be used in the diagnosis of DS by screening samples from the pregnant mother.

Accordingly, the present invention provides a method for diagnosing Down's syndrome (DS) in a human fetus comprising determining the amount of C1 inhibitor in a sample from the pregnant female carrying the fetus.

Such a method may further comprise comparing the amount of C1 inhibitor in the sample with the amount of C1 inhibitor in: (a) a sample from a non-pregnant female; and/or (b) a sample from a pregnant female carrying a fetus that does not have DS. An increased amount of C1 inhibitor in the sample from the pregnant female carrying the fetus of interest when compared to the sample from a non-pregnant female and/or from a pregnant female carrying a non-DS fetus may indicate that the fetus of interest is a DS fetus.

The level of C1 inhibitor may be determined by a method comprising triple quadrupole mass spectrometry; mass spectrometry; an Enzyme-linked Immunosorbant Assay (ELISA); and/or immunofluorescence.

Such methods may further comprise detecting or determining the amount of one or more additional biomarkers for DS. The one or more additional biomarker for DS may be selected from complement C3, transthyretin, serum amyloid protein (SAP), the β subunit of human chorionic gonadotrophin (β-hCG), pregnancy-associated plasma protein A (PAPP-a), alphafetoprotein (AFP), unconjugated estriol (uE3) and Inhibin-A.

The present inventors have also developed a diagnostic test for DS that can be applied to both C1 inhibitor and other DS biomarkers and which can be carried out in any trimester of pregnancy. This test is simple, non-invasive, rapid and cost effective test, uses equipment that is already available in every large hospital in the UK and fulfils the NSC requirements for DSS. This diagnostic test offers increased sensitivity and specificity over the tests presently available, requires a smaller sample volume and offers cost benefits over the known tests.

Accordingly, the present disclosure provides a method for diagnosing DS in a human fetus comprising determining the amount of at least one biomarker for DS in a sample from the pregnant female carrying the fetus using triple quadrupole mass spectrometry. The at least one biomarker for DS of the disclosure may be selected from C1 inhibitor, SAP, complement C3, transthyretin, β-hCG, PAPP-a, AFP, uE3 and Inhibin-A. The biomarker is C1 inhibitor. The triple quadrupole mass spectrometry may be used to conduct multiple reaction monitoring (MRM) peptide analysis of the at least one biomarker for DS.

Such a method may further comprise comparing the amount of the at least one biomarker for DS in said sample with the amount of the biomarker in: (a) a sample from a non-pregnant female; and/or (b) a sample from a pregnant female carrying a fetus that does not have DS. A difference in the presence of, or amount of, the biomarker may indicate that the fetus of interest is a DS fetus. For example, an increased amount of C1 inhibitor in the sample from the pregnant female carrying the fetus of interest when compared to the sample from a non-pregnant female and/or from a pregnant female carrying a non-DS fetus may indicate that the fetus of interest is a DS fetus.

In the triple quadrupole mass spectrometry methods of the invention, the amount of at least one biomarker for DS that is C1 inhibitor may be determined by comparing the amount of at least one marker peptide for said biomarker within the sample with a known amount of at least one deuterated standard peptide derived from said biomarker. Preferably in such a method, the biomarker is C1 inhibitor and at least one deuterated standard peptide comprises an amino acid sequence specific only for C1 Inhibitor. In a more preferred embodiment, the deuterated standard peptide that is specific for C1 inhibitor is FQPTLLTLPR.

In the diagnostic methods of the invention, the pregnant female may be in the first or second trimester of pregnancy

In the diagnostic methods of the invention, the sample may be a sample of blood, serum or plasma from the pregnant female. The sample may be collected on a Guthrie card. The volume of the sample may be less than 50 µl.

The present disclosure also provides test kits for use in accordance with the diagnostic methods of the invention. The disclosure provides a test kit for use in a triple quadrupole mass spectrometry method as described herein, said kit comprising at least one deuterated standard peptide derived from at least one biomarker for DS and instructions for carrying out a method of diagnosing DS using triple quadrupole mass spectrometry. The one or more additional biomarker for DS of the disclosure may be selected from C1 inhibitor, SAP, complement C3, transthyretin, β-hCG, PAPP-a, AFP, uE3 and Inhibin-A. The test kits of the disclosure may be for use in a method of MRM peptide analysis by triple quadrupole mass spectrometry and may comprise instructions for carrying out a method of diagnosing DS using MRM peptide analysis by triple quadrupole mass spectrometry. Preferably, the one or more additional biomarker for DS is or comprises C1 inhibitor. The at least one deuterated standard peptide may comprise a specific peptide amino acid sequence to C1 inhibitor, preferably FQPTLLTLPR.

### Description of the Figures

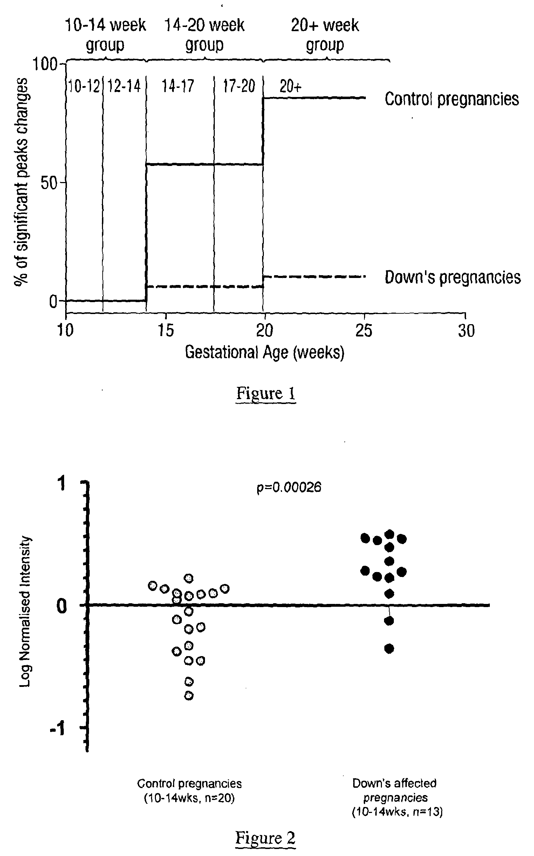

**Figure 1** shows overall changes in the maternal plasma proteome and how Gestational Age (GA) groups were defined. All peaks/proteins detected by biomarker wizard software (Ciphergen) were analysed for significant differential expression of total peak volume. Many subtle but significant changes were detected throughout gestational age. The chart shows overall change (differentially expressed proteins' (p<0.05)) as a percentage from the total no of detected proteins (100%) when compared with the subsequent increasing gestational age period i.e. initially a normal 10-12 wk group showed no changes compared with a 12-14 wk group therefore these GA groups were grouped together and compared with a 14-17wk group where changes were observed therefore this defined the 10-14wk first trimester group and subsequent comparisons defined the other GA groups. Normal pregnancy shows a lot of changes occurring from 14 wks onwards which is the time that the second trimester begins and further changes occur from 20 wks. This information was used to define the gestational age groups for comparison with DS pregnancies. When comparing DS pregnancies using these defined GA groups there is less of a degree of significant change occurring in the maternal plasma proteome. Bold line represents control group change. Dotted line represents DS group change. Ctl 10-14wk n=20, DS 10-14wok n=13, Ctl 14-20wk n=34, DS 14-20wk n=17, Ctl 20+ n=7 DS 20+ n=5.
**Figure 2****.** Plot of log normalised peak intensity of the potential biomarker of m/z 100.3 kDa detected in maternal plasma samples from control and Down's affected pregnancies. SELDI plot of log normalised peak intensity of first trimester maternal plasma samples. Control n=20, 0.75±0.168 vs DS n=13, 1.02 ±0.18 p=0.00026.

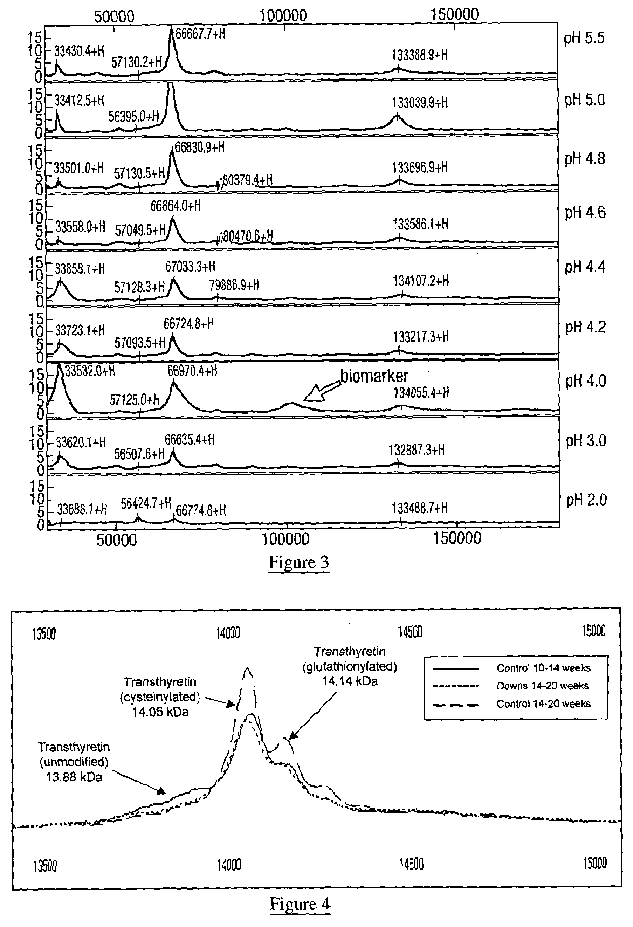

**Figure 3****.** SELDI TOF MS spectra of pH elutions from a ProteinChip Q preparative column demonstrating the enrichment of the potential plasma protein biomarker (C1-protein inhibitor) in the pH 4 fraction.
**Figure 4****.** Representative spectra of maternal plasma transthyretin from control and Down's affected pregnancies. Moore *et al.,* have described previously that transthyretin can exist in the maternal plasma as unmodified (13.88 kDa), cysteinylated (14.05 kDa) and gluthionylated (14.14 kDa) isoforms. An increase in the unmodified form of the transthyretin are observed at 14-20 weeks in the Down's affected pregnancies in association with a decrease in both post-translationally modified isoforms.

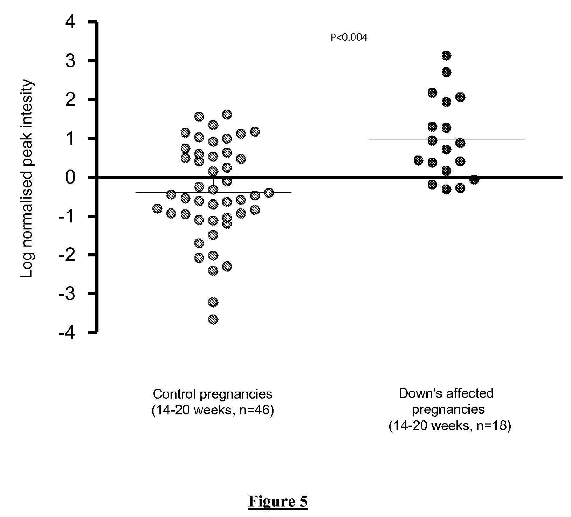

**Figure 5****.** Plot of log normalised peak intensity of the potential biomarker of m/z 23.5 kDa detected in maternal plasma samples from control and Down's affected pregnancies. 23.412 kDa protein SELDI plot of log normalised peak intensity of 14 - 20 week maternal plasma samples. Control n=28, 0.17(0.12) vs DS n=10, 0.47(0.36) p=0.004.

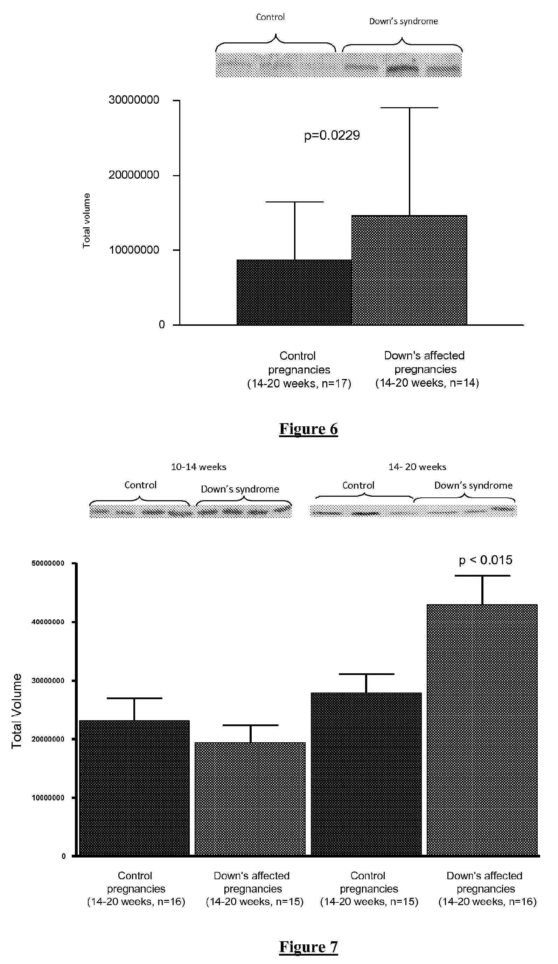

**Figure 6****.** Western blot and scanning densitometry analyses (mean ± SEM) for mass value 100,332 daltons demonstrating a 1.7 fold increase of C1 plasma protease inhibitor in maternal plasma of Down's affected pregnancies at 10-14 weeks gestation compared with normal first trimester pregnancies. Quantification of protein content of C1 inhibitor using scanning densitometry. Each bar represents the mean ± standard deviation (SD) of 17. Control and 14 DS samples measured in duplicate (p=0.0229). The panel above shows representative C1 inhibitor protein bands from 0.75ul of plasma from women with normal and DS pregnancies which were separated by gel electrophoresis and blotted with the optimised dilution of antibody.
**Figure 7****.** Western blot analysis and scanning densitometry showing a 1.54-fold increase of SAMP in the second trimester maternal plasma of Down's syndrome affected pregnancies (mean ± SEM). No significant difference in SAMP levels were observed during the 10-14 week analyses, indicating SAMP is only a good 2nd trimester marker for Down's syndrome. Each bar represents the mean ± SD of 16 Ctl-1 (control 10 - 14 weeks), 17 Ctl-2 (control 14 - 20 weeks), 15 T21-1 (DS 10 - 14 weeks) and 16 T21-2 (DS 14 - 20 weeks) samples measured in duplicate * p<0.015. The panel above shows representative SAMP protein bands from 0.75ul of plasma from women with normal and DS pregnancies which were separated by gel electrophoresis and blotted with the optimised dilution of antibody.

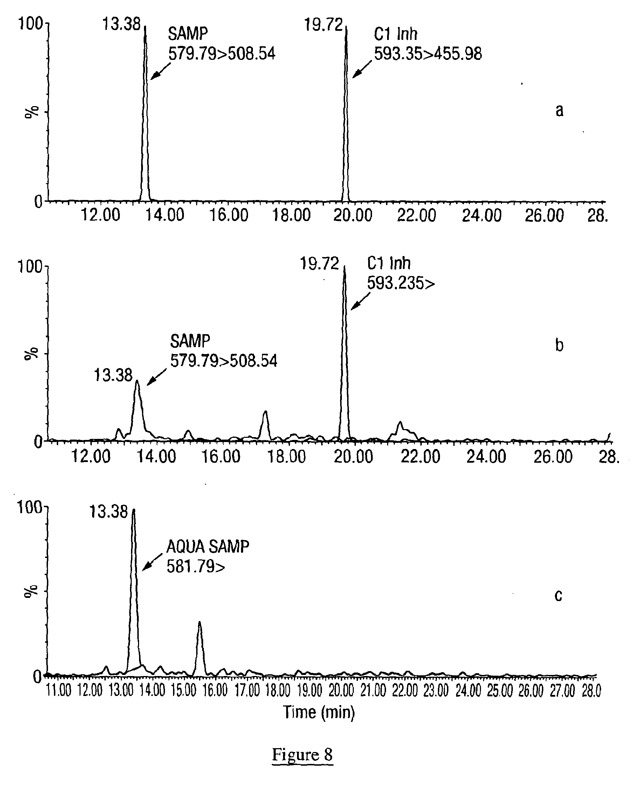

**Figure 8** shows MRM chromatograms from triple quadrupole mass spectrometry method analyses of control plasma. Figure 1a shows synthesised SAMP 579.79 and C1 inhibitor 593.35 peptides that were used for optimisation. Figure 1b shows endogenous peptides detected in 5µl of trypsin digested plasma. Figure 1c shows the digested internal standard (AQUA SAMP) peptide spiked in plasma.

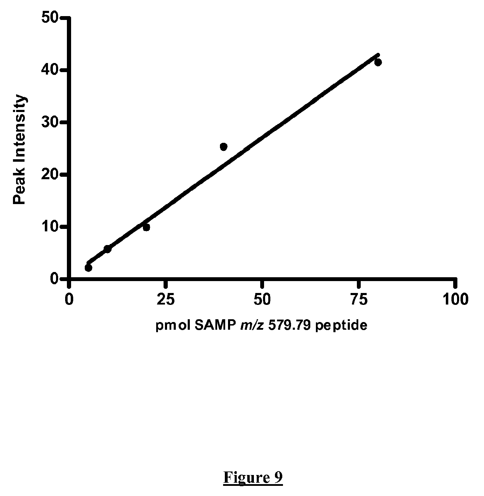

**Figure 9** shows a standard curve of duplicate samples of 5ml of plasma spiked with increasing amounts (pmols) of custom synthesised SAMP 579.79 peptide (Genscript). Endogenous SAMP 579.79 was subtracted from the peak intensity (r²= 0.983).

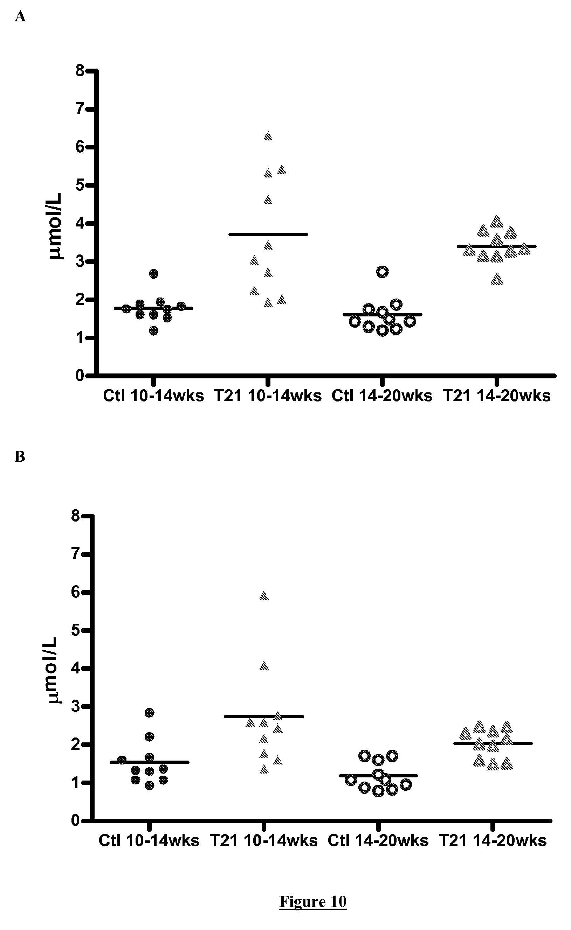

**Figure 10A** shows the average of duplicate mmol/L values of endogenous SAMP peptide plotted for each group. Native SAMP peptide is ratioed to internal AQUA peptide standard in 5ml of trypsin digested whole plasma. Significance according to students t-test. A 2.1 fold increase in DS 10 - 14 weeks (p<0.001) and a 2.2 fold increase is observed in the DS 14 - 20weeks group (p<0.0001).
**Figure 10B** shows the average of duplicate mmol/L values of endogenous C1 Inhibitor peptide plotted for each group. Native C1 Inhibitor peptide is ratioed to internal AQUA peptide standard in 5ml of trypsin digested whole plasma. Significance according to students t-test. A 1.8 fold increase (p<0.019) is seen for DS 10 - 14 weeks group and a 1.7 fold increase in DS 14 - 20 weeks (p< 0.0001).

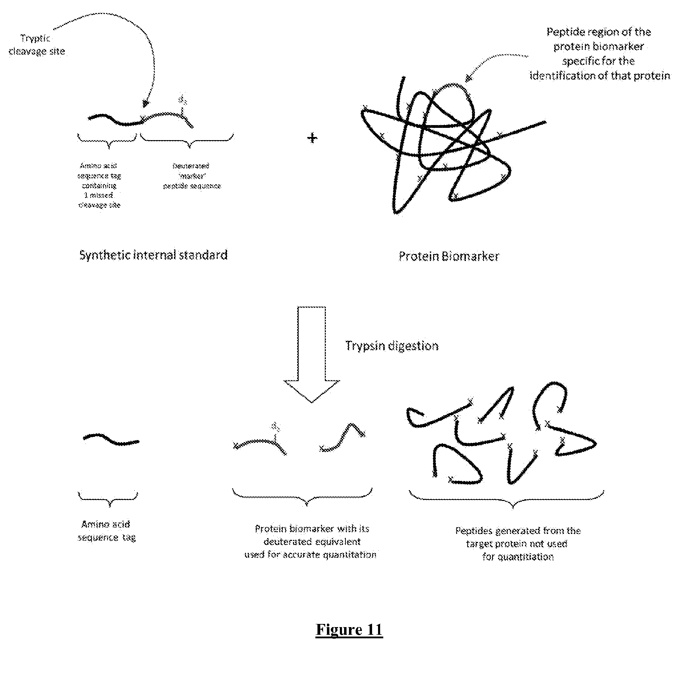

**Figure 11** shows a schematic representation and work-flow summarising the methods used for SRM-based quantitation of SAMP and C1 inhibitor.

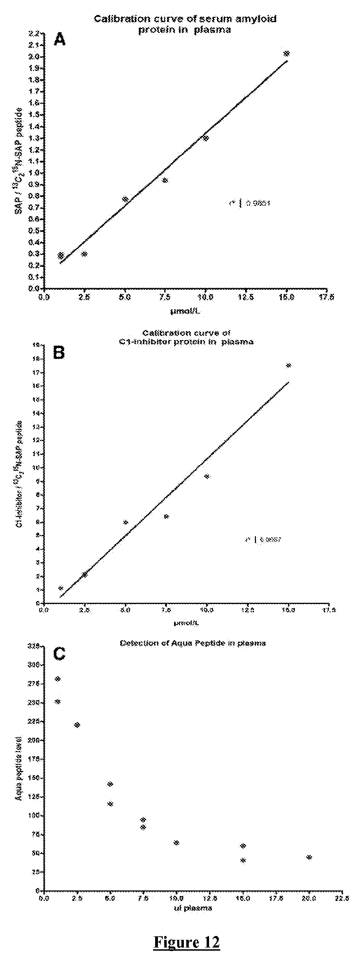

**Figure 12** **-** (A) Standard curve of the marker peptides for SAP (m/z 578.97) and (B) C1-inhibitor (m/z 593.54) spiked into a plasma digest and quantitated using the SAP Aqua™ peptide VGEYSL[C13N15]YIGR (C) demonstrates the effect of ion suppression of increasing amounts of plasma on the response of the SAP Aqua™ peptide.

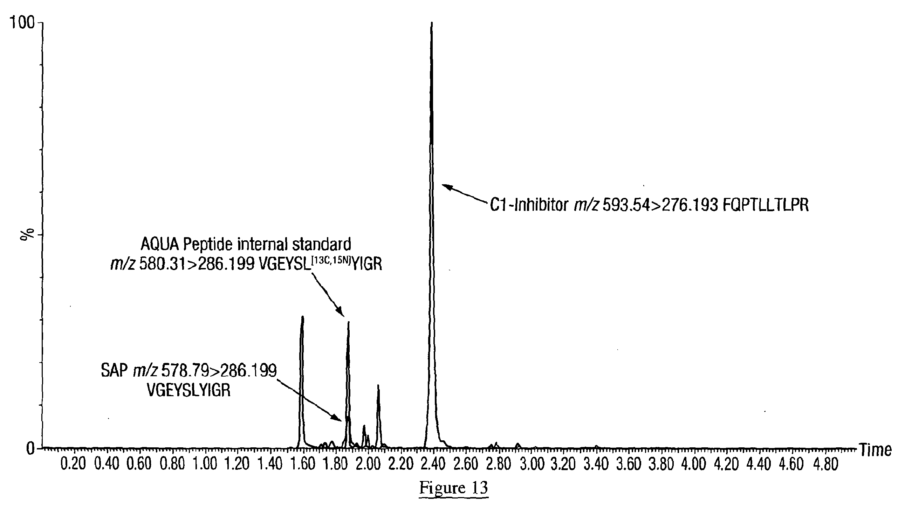

**Figure 13** **-** Typical HPLC-MS/MS chromatogram demonstrating the 5min analysis time of SAP and C1-inhibitor protein from 5 µl of plasma. The SRM transitions for SAP, AQUA™ peptide internal standard and C1-inhibitor are indicated.

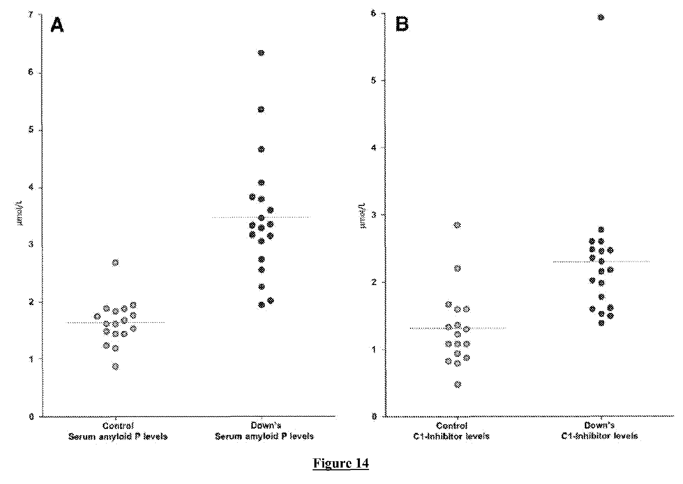

**Figure 14** **-** Graph showing the maternal plasma levels of (A) SAP levels (µmol/l), control 1.64±0.39, (SD) Downs, 3.46±1.09 (SD) p=0.0001 and (B) C1-plasma inhibitor (µmol/l), control 1.31±0.14, Downs 2.30±0.22, p=0.0008.

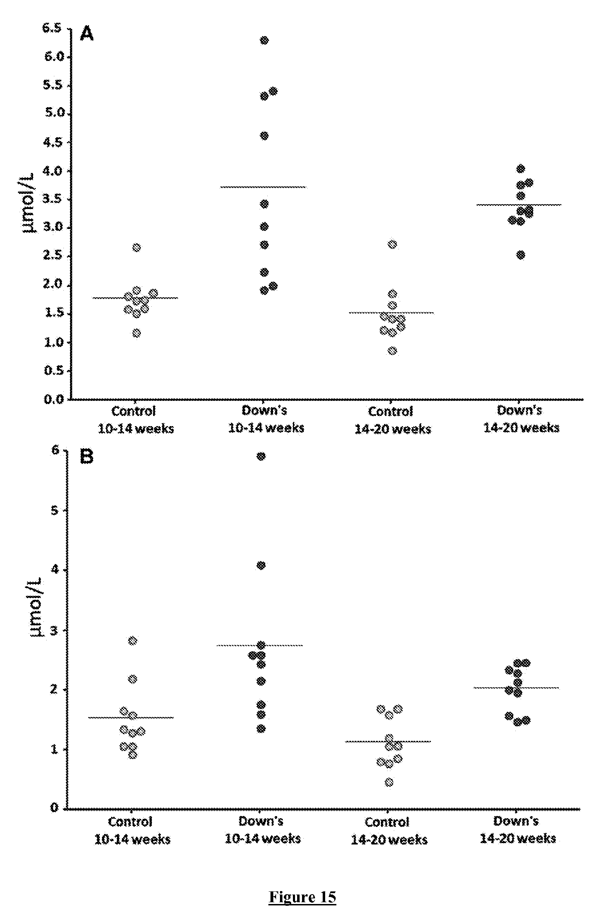

**Figure 15** **-** SRM assay of maternal plasma for (A) SAP levels (µmol/l) in first trimester (10-14 weeks), control 1.78±0.38 (SD), Downs 3.78±1.60, (SD), p=0.0015 and second trimester (14-20 weeks) control 1.53±0.51 (SD), Downs 3.41±0.43, (SD) p=0.0001; and (B) C1-plasma inhibitor (µmol/l) in 10-14 weeks, control 1.54±0.59 (SD), Downs 2.74±1.36, (SD) p=0.019 and 14-20 weeks, control 1.14±0.42 (SD), DS 2.03±0.39 (SD), p=0.0001. n=10 for all groups.

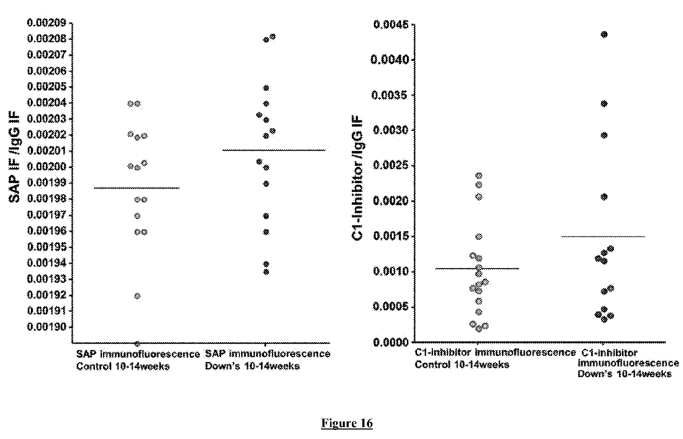

**Figure 16** **-** A graphical representation of maternal serum amyloid P and C1-inhibitor protein levels, in the circulation of control and Downs syndrome affected pregnancies (10-14 weeks gestation). SAP and C1-inhibitor were measured using an in-house developed immunofluorescence method and standardised against plasma IgG levels.

### Detailed Description of the Invention

The present invention provides methods for the diagnosis of Down's Syndrome (DS) in a fetus. These methods involve determining the amount of a biomarker for DS in a sample from the pregnant female carrying the fetus of interest. The biomarker for DS is plasma protease C1 inhibitor (C1 inhibitor). In some embodiments of the invention, the methods use triple quadrupole mass spectrometry. The methods of the invention may use MRM peptide analysis by triple quadrupole mass spectrometry.

### C1 inhibitor

C1 inhibitor is the main regulator of the early activation steps in the classical complement pathway. Levels of C1 inhibitor are known to be lower in pregnant females compared to non-pregnant females and genetic C1 inhibitor deficiency has been associated with complications during pregnancy.

The Inventors have now found that the level of C1 inhibitor in pregnant females may be used to aid the diagnosis of a Down's syndrome (DS) affected fetus. In particular, the Inventors have found that the level of plasma C 1 inhibitor is significantly elevated in DS affected pregnancies (see Example 1). It is possible to provide a method for diagnosing DS in a human fetus comprising determining the amount of C1 inhibitor in a sample obtained from the pregnant female carrying the fetus. Thus, the diagnosis of a DS affected fetus may be achieved by determining the amount of C1 inhibitor in a sample obtained from the pregnant female carrying the fetus and thereby diagnosing DS in the fetus. An increased level of C1 inhibitor in the sample, such as an increased level when compared to the level in a sample from a non-pregnant female or a sample from a female with a non-DS pregnancy, may reflect the presence of DS in the fetus.

The C1 inhibitor may have the amino acid sequence of the C1 inhibitor protein with UniProt Accession No. P05155.

The female may be in the first trimester or in the second trimester.

The amount of C1 inhibitor in a sample encompasses the mass of C1 inhibitor in the sample, the molar amount of C1 inhibitor in the sample, the concentration of C1 inhibitor in the sample, the molarity of C1 inhibitor in the sample. This amount may be given in any appropriate units. For example, the concentration of C1 inhibitor may be given in pg/ml, ng/ml or µg/ml. The mass of C1 inhibitor may be given in pg, ng or µg.

The amount of C1 inhibitor may be measured directly. For example, the C1 inhibitor itself may be detected in the sample and the amount of the C1 inhibitor in the sample may be assessed.

The amount of the C1 inhibitor may be measured indirectly. For example, an agent or label that binds to C1 inhibitor may be used to detect and/or quantify the C1 inhibitor. The amount of that agent or label may then be assessed as an indicator of the amount of C1 inhibitor in the sample.

The amount of the C1 inhibitor in a sample of interest may be compared with the level of C1 inhibitor in another sample as described herein. In such a method, the actual amount of the C1 inhibitor, such as the mass, molar amount, concentration or molarity of the C1 inhibitor in the samples may be assessed. The amount of C1 inhibitor in the samples may be measured directly or indirectly as described herein. The amount of C1 inhibitor may be compared with that in another sample without quantifying the mass, molar amount, concentration or molarity of the C1 inhibitor. Thus, the amount of C1 inhibitor in a sample according to the invention may be assessed as a relative amount, such as a relative mass, relative molar amount, relative concentration or relative molarity of the C1 inhibitor based on a comparison between two or more samples.

The amount of C1 inhibitor in a sample obtained from the pregnant female carrying the fetus may be determined using any appropriate technique. Standard methods known in the art may be used to assay the amount of C1 inhibitor. These methods may involve using an agent for the detection of C1 inhibitor and/or an agent for the determination of the amount of C1 inhibitor. Any method that allows for the detecting of C1 inhibitor and the quantification, or relative quantification of the C1 inhibitor may be used. The presence and/or amount of C1 inhibitor may be determined using techniques that involve binding an antibody or other ligand to the C1 inhibitor. Such an antibody or other ligand may be labelled and that label may be detected to detect and/or quantify the C1 inhibitor in the sample. Such techniques may include Enzyme-linked Immunosorbant Assay (ELISA) and immunofluorescence. The amount of C1 inhibitor may be determined using mass spectrometry. In a preferred embodiment, the amount of C1 inhibitor is determined using triple quadrupole mass spectrometry. MRM peptide analysis may be carried out by triple quadrupole mass spectrometry.

Agents for the detection of or for the determination of the amount of C1 inhibitor may be used to determine the amount of C1 inhibitor in a sample obtained from the pregnant female carrying the fetus. Such agents typically bind to C1 inhibitor. Such agents may bind specifically to C1 inhibitor. The agent for the detection of or for the determination of the amount of C1 inhibitor may be an antibody or other binding agent specific for C1 inhibitor. By specific, it will be understood that the agent or antibody binds to the molecule of interest, in this case C1 inhibitor, with no significant cross-reactivity to any other molecule, particularly any other protein. For example, an agent or antibody that is specific for C1 inhibitor will show no significant cross-reactivity with human neutrophil elastase. Cross-reactivity may be assessed by any suitable method. Cross-reactivity of an agent or antibody for C1 inhibitor with a molecule other than C1 inhibitor may be considered significant if the agent or antibody binds to the other molecule at least 5%, 10%, 15%, 20%, 25%, 30%, 35%, 40%, 45%, 50%, 55%, 60%, 65%, 70%, 75%, 80%, 85%, 90% or 100% as strongly as it binds to C1 inhibitor. An agent or antibody that is specific for C1 inhibitor may bind to an other molecule such as human neutrophil elastase at less than 90%, 85%, 80%, 75%, 70%, 65%, 60%, 55%, 50%, 45%, 40%, 35%, 30%, 25% or 20% the strength that it binds to C1 inhibitor. Preferably, the agent or antibody binds to the other molecule at less than 20%, less than 15%, less than 10% or less than 5%, less than 2% or less than 1% the strength that it binds to C1 inhibitor.

An antibody used in the method of the invention may be a whole antibody or a fragment thereof which is capable of binding to C1 inhibitor e.g. Fab or F(ab)2 fragments. The antibody may be monoclonal or polyclonal. The antibody may be produced by any suitable method known in the art. For example, polyclonal antibodies may be obtained by immunising a mammal, typically a rabbit or a mouse, with C1 inhibitor under suitable conditions and isolating antibody molecules from, for example, the serum of said mammal. Monoclonal antibodies may be obtained by hybridoma or recombinant methods.

Hybridoma methods may involve immunising a mammal, typically a rabbit or a mouse, with C1 inhibitor under suitable conditions, then harvesting the spleen cells of said mammal and fusing them with myeloma cells. The mixture of fused cells is then diluted and clones are grown from single parent cells. The antibodies secreted by the different clones are then tested for their ability to bind to C1 inhibitor, and the most productive and stable clone is then grown in culture medium to a high volume. The secreted antibody is collected and purified.

Recombinant methods may involve the cloning into phage or yeast of different immunoglobulin gene segments to create libraries of antibodies with slightly different amino acid sequences. Those sequences which give rise to antibodies which bind to C1 inhibitor may be selected and the sequences cloned into, for example, a bacterial cell line, for production.

Typically the antibody is a mammalian antibody, such as a primate, human, rodent (e.g. mouse or rat), rabbit, ovine, porcine, equine or camel antibody. The antibody may be a camelid antibody or shark antibody. The antibody may be a nanobody. The antibody can be any class or isotype of antibody, for example IgM, but is preferably IgG. The antibody may be a humanised antibody.

The antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities, e.g. that can bind the same or different polymorphisms. The antibody may be a bispecific antibody which is able to bind to two different antigens, typically any two of the polymorphisms mentioned herein. The antibody may be a 'diabody' formed by joining two variable domains back to back. In the case where the antibodies used in the method are present in any of the above forms which have different antigen binding sites of different specificities then these different specificities are typically to polymorphisms at different positions or on different proteins. In one embodiment the antibody is a chimeric antibody comprising sequence from different natural antibodies, for example a humanised antibody.

Methods to assess an amount of C1 inhibitor may involve contacting a sample with an agent or antibody capable of binding specifically to C1 inhibitor. Such methods may include dipstick assays and Enzyme-linked Immunosorbant Assay (ELISA). Other immunoassay types may also be used to assess C1 inhibitor amounts. Typically dipsticks comprise one or more antibodies or proteins that specifically bind C1 inhibitor. If more than one antibody is present, the antibodies preferably have different non-overlapping determinants such that they may bind to C1 inhibitor simultaneously.

ELISA is a heterogeneous, solid phase assay that requires the separation of reagents. ELISA is typically carried out using the sandwich technique or the competitive technique. The sandwich technique requires two antibodies. The first specifically binds C1 inhibitor and is bound to a solid support. The second antibody is bound to a marker, typically an enzyme conjugate. A substrate for the enzyme is used to quantify the C1 inhibitor -antibody complex and hence the amount of C1 inhibitor in a sample. The antigen competitive inhibition assay also typically requires a C1 inhibitor -specific antibody bound to a support. A C1 inhibitor -enzyme conjugate is added to the sample (containing C1 inhibitor) to be assayed. Competitive inhibition between the C1 inhibitor -enzyme conjugate and unlabeled C1 inhibitor allows quantification of the amount of C1 inhibitor in a sample. The solid supports for ELISA reactions preferably contain wells.

Antibodies capable of binding specifically to C1 inhibitor may be used in methods of immunofluorescence to detect the presence of C1 inhibitor and hence in methods of diagnosing DS according to the present invention.

The present invention may also employ methods of determining the amount of C1 inhibitor that do not comprise antibodies. High Performance Liquid Chromatography (HPLC) separation and fluorescence detection is preferably used as a method of determining the amount of C1 inhibitor. HPLC apparatus and methods as described previously may be used (Tsikas D et al. J Chromatogr B Biomed Sci Appl 1998; 705: 174-6) Separation during HPLC is typically carried out on the basis of size or charge. Prior to HPLC, endogenous amino acids and an internal standard L-homoarginine are typically added to assay samples and these are phase extracted on CBA cartridges (Varian, Harbor City, CA). Amino acids within the samples are preferably derivatized with *o-*phthalaldehyde (OPA). The accuracy and precision of the assay is preferably determined within quality control samples for all amino acids.

Other methods of determining the amount of C1 inhibitor that do not comprise antibodies include mass spectrometry. Mass spectrometric methods may include, for example, matrix-assisted laser desorption/ionization mass spectrometry (MALDI MS), surface-enhanced laser desorption/ionization mass spectrometry (SELDI MS), time of flight mass spectrometry (TOF MS) and liquid chromatography mass spectrometry (LC MS). In a preferred embodiment, triple quadrupole mass spectrometry is used to determine the amount of C1 inhibitor. MRM peptide analysis by triple quadrupole mass spectrometry may be used to determine the amount of C1 inhibitor.

### Other biomarkers for DS

The methods of the invention comprise detecting or determining the amount of one or more biomarkers for DS. A biomarker is a characteristic or molecule that can be objectively measured and evaluated as an indicator of a biologic processes or state, such as DS. Different biomarkers for DS may increase or decrease in amount when DS is present.

The biomarker is C1 inhibitor. The amount of C1 inhibitor in a sample from a pregnant female may be used to determine whether the fetus carried by that female has DS. As discussed above, the amount of C1 inhibitor may be assessed by any known method. An increased level of C1 inhibitor as described herein indicates that the fetus carried by the female may have DS.

According to the present invention, a method for diagnosing DS may comprise determining the amount of C1 inhibitor in a sample from a pregnant female and also detecting or determining the amount of one or more additional biomarkers.

Additional biomarkers for DS include serum amyloid protein (SAP), complement C3, complement C3 alpha chain, transthyretin, the β subunit of human chorionic gonadotrophin (β-hCG), pregnancy-associated plasma protein A (PAPP-a), alphafetoprotein (AFP), unconjugated estriol (uE3) and Inhibin-A.

A method for diagnosing DS may comprise determining the amount of one or more of those additional biomarkers alone, such as complement C3 or transthyretin. That is, a method described herein which includes the detection or quantification of C1 inhibitor may be used using such an additional biomarker in place of the C1 inhibitor.

As reported in Example 1, complement C3 or complement C3 alpha chain may be detected in order to diagnose DS. A reduced level of complement C3 or complement C3 alpha chain is seen in samples from pregnant females carrying DS pregnancies. Such a reduced level may therefore be used to diagnose DS. The level of complement C3 or complement C3 alpha chain may be assessed during the second trimester (14-20 weeks).

Transthyretin in maternal plasma normally increases between the 10-14 week period of pregnancy and the 14-20 week period. This increase was not seen in DS pregnancies (see Example 1). A comparison of transthyretin levels in maternal plasma between the 10-14 week period and the 14-20 week period may therefore assist in diagnosing DS. In a normal pregnancy, an increase in transthyretin would be expected, so the lack of an increase may be used to diagnose DS. Further analysis of the different glycofiorms of transthyretin may also be used. As shown in Table 1, a decrease in the amount of cysteinylated (14.05 kDa), or gluthionylated (14.14 kDa) transthyretin or an increase in the amount of unmodified (13.88 kDa) transthyretin may be used to diagnose DS. In particular, these changes may be assessed during the second trimester, such as between week 14 and week 20 of pregnancy.

The one or more additional biomarkers may be detected or the amount of the one or more additional biomarkers determined using any of the techniques disclosed herein for the determination of the amount of C1 inhibitor. Agents or antibodies specific for the one or more additional biomarkers may be used in such techniques. The one or more additional biomarkers may, for example, be detected, or the amount of the one or more additional biomarkers determined using ELISA, immunofluorescence, HPLC, or mass spectrometry. Mass spectrometric methods may include, for example, MALDI MS, SELDI MS, TOF MS, LC MS and triple quadrupole mass spectrometry. MRM peptide analysis by triple quadrupole mass spectrometry may be used to determine the amount of the one or more additional biomarkers.

The one or more additional biomarkers may be detected or the amount of the one or more additional biomarkers determined in the same sample obtained from the pregnant female undergoing DSS in which the amount of C1 inhibitor is determined.

The one or more additional biomarkers may be detected or the amount of the one or more additional biomarkers determined at the same time as the amount of C1 inhibitor is determined. Alternatively, the one or more additional biomarkers may be detected or the amount of the one or more additional biomarkers determined before or after the amount of C1 inhibitor is determined.

According to the present disclosure, a method for diagnosing DS may comprise determining the amount of at least one biomarker for DS in a sample from a pregnant female carrying the fetus of interest. In this method the amount of the biomarker is determined using triple quadrupole mass spectrometry. Thus, the diagnosis of a DS affected fetus may be achieved by determining the amount of at least one biomarker in a sample obtained from the pregnant female carrying the fetus and thereby diagnosing DS in the fetus.

The biomarker for DS to be used in such a method may be any of the biomarkers for DS described herein. For example, the biomarker for DS may be C1 inhibitor. The biomarker for DS may be selected from serum amyloid protein (SAP), the β subunit of human chorionic gonadotrophin (β-hCG), pregnancy-associated plasma protein A (PAPP-a), alphafetoprotein (AFP), unconjugated estriol (uE3) and Inhibin-A.

The "amount" of a biomarker for DS will be understood to have the equivalent meaning as the "amount" of C1 inhibitor as discussed above. Any of the meanings or definitions of an amount discussed above in relation to C1 inhibitor also apply to other biomarkers of DS as discussed herein. The amount of any biomarker of DS may be determined using any of the methods described above for determining the amount of C1 inhibitor.

The discussion above in relation to the particular biomarker for DS, C1 inhibitor also applies to other biomarkers of DS. Agents for the detection of a biomarker of DS may be the same types of agents described above in relation to the particular biomarker for DS, C1 inhibitor. Equivalent agents may be used to detect other biomarkers of DS. For example, any antibody to a biomarker of DS may be used in the same way described above for antibodies to C1 inhibitor. The same types of antibodies described above in relation to C1 inhibitor may be used to detect other biomarkers of DS. Such agents or antibodies may be specific to a biomarker of DS as defined above in relation to the specific biomarker C1 inhibitor. The methods described above for the detection or determining the amount of C1 inhibitor may be used to detect or determine the amount of other biomarkers of DS.

### Simple obtained from a pregnant female,

The invention is typically carried out *in vitro* on a sample obtained from a pregnant female. The sample typically comprises a body fluid of the pregnant female. The sample is not obtained from the fetus. The sample typically does not comprise a body fluid of the fetus.

The sample may be or may have been obtained from the pregnant female at any stage during pregnancy. Preferably the sample is obtained from a pregnant female in the first or second trimester of pregnancy. Preferably, the sample is obtained from a pregnant female in the late first or early second trimester of pregnancy. Typically the sample if obtained from a pregnant female at a stage of pregnancy from about 10 to 14 or 14 to 20 weeks gestation. The sample may be obtained from a pregnant female at a stage of pregnancy at about 10, 11, 12, 13, 14, 15, 16, 17, 18, 19 or 20 weeks gestation.

The sample is preferably a sample of the blood, plasma or serum of the pregnant female. The sample may be less than 1 ml in volume. For example, the sample may be 5µl or less, 10µl or less, 20µl or less, 30µl or less, 40µl or less, 50µl or less, 60µl or less, 70µl or less, 80µl or less, 90µl or less, 100µl or less, 150µl or less, 200µl or less, 250µl or less, 300µl or less, 350µl or less, 400µl or less, 450µl or less or 500µl or less in volume. Preferably the sample is less than 500 µl, less than 250 µl, less than 100 µl or less than 50 µl. A blood, plasma or serum sample of such a volume may be taken on a blood sample card or Guthrie card, which does not require the presence of a trained phlebotomist.

Alternatively, the sample may be 1 ml or more in volume. For example, the sample may be up to 1ml, up to 2ml, up to 3ml, up to 4ml, up to 5ml, up to 6ml, up to 7ml, up to 8ml, up to 9ml, up to 10ml, up to 15ml, up to 20ml or up to 25ml. A blood, plasma or serum sample of ml volume may require the sample to be obtained by a phlebotomist.

The sample may be processed prior to being assayed, for example by centrifugation. The sample may also be stored prior to assay, for example below -80°C.

The sample obtained from a non-pregnant female or a pregnant female carrying a fetus that does not have DS may be obtained as outlined above.

When the amount of C1 inhibitor in the sample obtained from the pregnant female undergoing DSS is compared with the amount of C1 inhibitor in a sample from a non-pregnant female or a sample from a pregnant female carrying a fetus that does not have DS, the sample from the non-pregnant female or the sample from the pregnant female carrying a fetus that does not have DS is preferably a sample of the same body fluid and of the same volume.

### Comparative methods.

In the methods of the invention, the amount of a biomarker such as C1 inhibitor in a sample from the pregnant female carrying the fetus of interest may be compared with the amount of said biomarker in one or more other samples. This comparison may be with a control sample. A suitable control sample is from a comparable source, such as from another female or another pregnant female.

Where the control sample is from a pregnant female, that pregnant female is typically at an equivalent stage of pregnancy to the pregnant female undergoing DSS. An equivalent stage of pregnancy may be, for example the same trimester. Preferably the control pregnant female may be in the first or second trimester of pregnancy. Preferably, the control pregnant female is in the late first or early second trimester of pregnancy. Typically the control pregnant female is at a stage of pregnancy from about 10 to 14 or 14 to 20 weeks gestation. The control pregnant female may be at a stage of pregnancy at about 10, 11, 12, 13, 14, 15, 16, 17, 18, 19 or 20 weeks gestation. Preferably the control pregnant female is carrying a fetus that does not have DS.

A suitable control sample is from a source that is not expected to have elevated levels of the biomarker such as C1 inhibitor. A suitable control sample may be a non-pregnant female or a pregnant female carrying a non-DS fetus. For example, the sample from the pregnant female carrying the fetus of interest may be compared with a sample from a non-pregnant female. The sample from the pregnant female carrying the fetus of interest may be compared with a sample from a pregnant female carrying a fetus that does not have DS.

In some embodiments, the control sample may be from a female at a different stage of pregnancy. For example, some biomarkers such as transthyretin may change in levels at different stages of pregnancy. A test sample taken at one stage of pregnancy may be compared against a control sample from a different stage of pregnancy. The control sample may be from the same pregnant female at a different stage of the same pregnancy or from a different pregnant female. A test sample taken during the second trimester may be compared with a control sample taken during the first trimester.

These methods allow for the comparison of the amount of biomarker such as C1 inhibitor in different samples. These methods allow for the determination of the relative amount of a biomarker such as C1 inhibitor in a sample from the pregnant female carrying the fetus of interest when compared to a control.

If the amount of a biomarker for DS such as C1 inhibitor in the sample from the pregnant female carrying the fetus of interest is greater than that in the control sample, such as a sample from a non-pregnant female, and/or a pregnant female carrying a non-DS fetus, this indicates that the fetus of interest is likely to be a DS fetus. This may indicate a diagnosis of DS for the fetus or may suggest the presence of DS. Such a diagnosis or suggestion may lead to further testing for DS.

Some biomarkers have a reduced level in DS. For those biomarkers, such as complement C3, if the amount of a biomarker in the sample from the pregnant female carrying the fetus of interest is lower than that in the control sample, such as a sample from a non-pregnant female, and/or a pregnant female carrying a non-DS fetus, this indicates that the fetus of interest is likely to be a DS fetus. This may indicate a diagnosis of DS for the fetus or may suggest the presence of DS. Such a diagnosis or suggestion may lead to further testing for DS.

If the amount of a biomarker for DS such as C1 inhibitor in the sample from the pregnant female carrying the fetus of interest is significantly greater than that in the control sample, such as a sample from a non-pregnant female, and/or a pregnant female carrying a non-DS fetus, this indicates that the fetus of interest is likely to be a DS fetus.

If the amount of a biomarker for DS such as complement C3 in the sample from the pregnant female carrying the fetus of interest is significantly lower than that in the control sample, such as a sample from a non-pregnant female, and/or a pregnant female carrying a non-DS fetus, this indicates that the fetus of interest is likely to be a DS fetus.

A significant increase or decrease may be assessed by standard statistical methods. For example, a biomarker for DS (such as C1 inhibitor) amount above the 95% confidence interval for the level in a control sample such as a pregnant female carrying a fetus that does not have DS may be associated with DS or an increased likelihood of DS.

According to the present invention, the increase or decrease in amount of at least one biomarker for DS which is associated with a risk of DS may be an increase or decrease of at least 1.5 fold and typically by at least 1.6 fold, 1.7 fold, 1.8 fold, 1.9 fold, 2 fold, 3 fold, 4 fold, 5 fold or 10 fold relative to the amount of the same biomarker(s) for DS in a control sample, such as a sample from a non-pregnant female, and/or the amount of the same biomarker(s) for DS in a sample from a pregnant female carrying a fetus that does not have DS.

The level of C1 inhibitor in a pregnant female carrying a fetus that does not have DS between 10 to 14 weeks gestation is typically between 1.25 µmol/L and 1.75 µmol/L. The level of C1 inhibitor in a pregnant female carrying a fetus that does not have DS between 14 to 20 weeks gestation is typically between 1.00 µmol/L and 1.50 µmol/L. The level of C1 inhibitor in a pregnant female carrying a fetus that does have DS between 10 to 14 weeks gestation is typically between 2.25 µmol/L and 3.25 µmol/L. The level of C1 inhibitor in a pregnant female carrying a fetus that does have DS between 14 to 20 weeks gestation is typically between 1.75 µmol/L and 2.25 µmol/L. The mean ± SEM level of C1 inhibitor in a normal pregnant female between 10-14 weeks gestation may be 1.54 ± 0.18 µmol/L and 1.18 + 0.11 µmol/L between 14-20weeks. In a DS affected pregnancy the mean C1 inhibitor levels may be 2.74 ± 0.43 between 10-14 weeks and 2.03 + 0.12 between 14-20 weeks.

According to the present invention, the increase in C1 inhibitor amount which is associated with a risk of DS is an increase of at least 1.5 fold and typically at least 1.6 fold, 1.7 fold, 1.8 fold, 1.9 fold, 2 fold, 3 fold, 4 fold, 5 fold or 10 fold relative to the amount of C1 inhibitor in a sample from a non-pregnant female, and/or the amount of C1 inhibitor in a sample from a pregnant female carrying a fetus that does not have DS.

In order to take account of this variation, the amount of C1 inhibitor may be expressed as a multiple of the median for unaffected pregnancies of the same gestational age (MoM). The amount of other biomarkers for DS may be expressed in the same way. For example, if the median biomarker level is 2.5 µmol/L at 10 weeks, 5.0 µmol/L at 12 weeks and 10.0 µmol/L at 14 weeks, the biomarker level of a woman who is found to have 5.0 µmol/L biomarker at 10 weeks is twice that of the median (5.0/2.5) or 2.0 MoM. Similarly, the biomarker level of a woman who is found to have 5.0 µmol/L at 14 weeks is half that of the median (5.0/10.0) or 0.5 MoM.

According to the present invention, the amount of C1 inhibitor amount which is associated with a risk of DS may be expressed as an MoM of at least 1.5, at least 1.6, at least 1.7, at least 1.8, at least 1.9, at least 2.0, at least 3.0, at least 4.0, at least 5.0 or at least 10.0.

### Further diagnostic methods.

The methods of the invention allow for a diagnosis for DS to carry out on a sample from the pregnant female that is carrying the fetus. Such methods have the advantage that they are non-invasive to the fetus.

The methods of the invention may be used in combination with other methods for detecting or diagnosing DS.

These additional tests may be carried out at the same time, before or after the amount of the at least C1 inhibitor is determined. For example, if a fetus has been identified by a method of the present invention as having or likely to have DS, then this diagnosis may be confirmed by other methods.

Examples of such additional diagnostic tests that may be used are ultrasound, the cffDNA test, CVS and amniocentesis. Such other methods may involve detection of other biomarkers of DS.

### Triple quadrupole mass spectrometry

The present invention provides methods for diagnosing DS in a human fetus comprising determining the amount of C1 inhibitor in a sample from the pregnant female carrying the fetus using triple quadrupole tandem mass spectrometry HPLC, referred to herein as triple quadrupole mass spectrometry. The biomarker is C1 inhibitor. The diagnosis of a DS affected fetus may be achieved by determining the amount of at least one biomarker for DS in a sample obtained from the pregnant female carrying the fetus and thereby diagnosing DS in the fetus.

Tandem mass spectrometry, including triple quadrupole mass spectrometry is commonly used to quantatively measure small molecules such as metabolites and peptides. A proteolytic or chemical digest of the tissue that the protein is to be measured is first separated by HPLC and then peptides are delivered by electrospray into the mass spectrometer. The mass spectrometer can be used to detect known precursor peptide mass within the tissue digest in a specific manner. The known precursor peptide is then fragmented to create known product ions, thereby creating a unique fragment profile for that peptide and hence for the protein of interest. Consequently, it is possible to quantitatively measure the amount of the protein of interest.

Quadrupole mass analysers comprise four circular rods in a parallel arrangement. When a fixed direct current and an alternating radio frequency are applied to a quadrupole mass analyser, ions passing through the analyser are separated based on the stability of their trajectories in the oscillating fields. Only ions of a particular mass:charge ratio (m/z) will have a stable trajectory at a given radio frequency. The radio frequency may be varied to bring ions of different m/z into focus on a detector and thus build up a mass spectrum. Quadrupole mass analysers can be placed in tandem to enable them to perform fragmentation studies. The use of three quadrupole mass analysers in tandem is known as a triple quadrupole and enables ion fragmentation studies to be carried out.

Triple quadrupole mass spectrometry may be used to conduct multiple reaction monitoring (MRM) peptide analysis. MRM peptide analysis by triple quadrupole mass spectrometry may be used to determine the amount of at least one biomarker for DS in a sample from the pregnant female carrying the fetus.

The biomarker is C1 inhibitor. The diagnosis of a DS affected fetus may be achieved by determining the amount of at least one biomarker for DS in a sample obtained from the pregnant female carrying the fetus and thereby diagnosing DS in the fetus.

Phenylketonuria (PKU) testing is routinely carried out on newborn children in the UK. This testing is carried out using triple quadrupole mass spectrometry. Every large hospital in the UK that carries out the screening process for PKU has at least two triple quadrupole mass spectrometers, one of which is used routinely for screening purposes, the other of which is a back-up. The back-up triple quadrupole mass spectrometer is therefore generally under-used. The present invention provides a method for diagnosing DS using triple quadrupole mass spectrometry. The necessary equipment for DSS using the method of the invention is therefore already available, and often under-used, in hospitals that carry out routine screening of fetuses and newborns.

The method for diagnosing DS according to the present invention is also more sensitive and specific that the methods known in the art, as illustrated in the Examples. The method of diagnosing DS using triple quadrupole mass spectrometry according to the present invention may be 10%, 20%, 30%, 40%, 50%, 60%, 70%, 80%, 90%, 100%, 150%, 200%, 250%, 300%, 400% or 500% more sensitive than methods known in the art.

The amount of a biomarker for DS in a sample may be determined by comparison of the MRM chromatograms obtained from the sample with the peak obtained for a deuterated standard peptide derived from the same biomarker peptide which is analysed by triple quadrupole mass spectrometry.

A standard peptide is a polypeptide at least comprises the same amino acid sequence as a fragment of the biomarker that is produced when the biomarker is fragmented during mass spectrometric analysis. A standard peptide is at least selective for, and preferably specific to, the requisite biomarker, such that the standard peptide can be used to identify said biomarker and to confirm and/or quantify the presence of said biomarker within a sample to be tested. The detection of a standard peptide for a particular biomarker when a sample is analysed by mass spectrometry is determinative of the presence of the corresponding biomarker in the sample being tested.

Standard peptides may be a fragment of between 4 to 25 amino acids, such as 4 to 20, 5 to 20 or 5 to 10 amino acids from the amino acid sequence of the biomarker. A standard peptide may comprise at least 4, 5, 6, 7, 8, 9, 10, 11, 12, 13, 14, 15, 20 or 25 amino acids in sequence from the biomarker. A standard peptide may comprise up to 25, up to 20, up to 15, up to 12, up to 10 or up to 8 amino acids in sequence from the biomarker. The size of the standard peptide may have a practical upper limit in view of the use of tandem mass spectrometry, including triple quadrupole mass spectrometry. This will be dependent on the particular mass spectrometer used, but may be, for example, a maximum size of 1500Da, 2000 Da or 2500 Da.

Typically the preferred standard peptide or peptides for a biomarker are fragmented during triple quadrupole mass spectrometry into a single predominant daughter ion and produce the largest response compared to other standard peptides for the same biomarker as detected using triple quadrupole mass spectrometry. In other words, the preferred standard peptide or peptides for a biomarker have greater sensitivity and specificity than other standard peptides for the same biomarker. By greater sensitivity, it is meant that the preferred standard peptide or peptides can be used to detect lower levels of the biomarker than other standard peptides for the same biomarker. By greater specificity, it is meant that the presence of a preferred standard peptide or peptides is indicative of the presence of the biomarker of interest, for example C1 inhibitor, and not of any other molecule, particularly any other protein, especially any other biomarker for DS. The standard peptide may be selective for the biomarker of interest. That is, the standard peptide may be indicative of the presence of the biomarker of interest and capable of distinguishing between the presence of that biomarker and other molecules such as other proteins or other biomarkers that might be present in the sample. For example, where the biomarker is C1 inhibitor, a standard peptide will not be present in a test sample comprising SAMP and/or APF, but not C1 inhibitor.

The standard peptide may be produced synthetically or generated from a naturally occurring biomarker. For example, standard peptides may be produced digesting a biomarker with trypsin or other enzymes, including chymotrypsin, slymotrypsin, ARG-C, CNBr, EndoLys-C, S.Aureus pH8, S.Aureus pH4, Asp-N, V8 protease or non-specific proteases. Standard peptides may also be produced by non-enzymatic methods. Deuterated standard peptides are standard peptides into which at least one atom of deuterium, ²H, has been incorporated.

In a preferred embodiment, the at least one biomarker for DS is C1 inhibitor and the deuterated standard peptide is a standard peptide derived from C1 inhibitor. In a more preferred embodiment, the deuterated standard peptide derived from C1 inhibitor comprises an amino acid sequence selected from LYHAFSAMK, TNLESILSYPKDFTCVHQALK, GVTSVSQIFHSPDLAIR, TLYSSSPR, LLDSLPSDTR, LVLLNAIYLSAK, YPVAHFIDQTLK, LEDMEQALSPSVFK, FQPTLLTLPR, VTTSQDMLSIMEK and TLLVFEVQQPFLFVLWDQQHK. In a particularly preferred embodiment the standard peptide comprises or consists of the amino acid sequence FQPTLLTLPR.

Multiple deuterated standard peptides derived from a single biomarker for DS may be used for determining the level or concentration of the biomarker for DS contained in the sample. Deuterated standard peptides derived from a combination of two or more biomarkers for DS may be used to determine the level or concentration of the corresponding biomarkers within the sample. In a preferred embodiment, deuterated standard peptides derived from C1 inhibitor and one or more of SAP, complement C3, transthyretin, PAPP-A, free β-hCG, AFP, uE3 and Inhibin-A are used to determine the level or concentration of the corresponding biomarkers within the sample.

The standard peptide for AFP may comprise an amino acid sequence selected from YGHSDCCSQSEEGR, HNCFLAHK, HPFLYAPTILLWAAR, AENAVECFQTK, ESSLLNQHACAVMK, TFQAITVTK, VNFTEIQK, LVLDVAHVHEHCCR, GDVLDCLQDGEK, IMSYICSQQDTLSNK, GQCIIHAENDEKPEGLSPNLNR, DFNQFSSGEK, NIFLASFVHEYSR, HPQLAVSVILR, GYQELLEK, CFQTENPLECQDK, YIQESQALAK, LGEYYLQNAFLVAYTK, APQLTSSELMAITR, MAATAATCCQLSEDK, LLACGEGAADIIIGHLCIR, HEMTPVNPGVGQCCTSSYANR, RPCFSSLVVDETYVPPAFSDDK, DLCQAQGVALQTMK, QEFLINLVK, QKPQITEEQLEAVIADFSGLLEK and CCQGQEQEVCFAEEGQK. In a preferred embodiment the standard peptide for AFP comprises or consists of the amino acid sequence AENAVECFQTK.

The standard peptide for free β-hCG may comprise an amino acid sequence selected from CRPINATLAVEK, EGCPVCITVNTTICAGYCPTMTR, VLQGVLPALPQVVCNYR, GVNPVVSYAVALSCQCALCR, STTDCGGPK, DHPLTCDDPR, FQDSSSSK, APPPSLPSPSR and LPGPSDTPILPQ. In a preferred embodiment the standard peptide for free β-hCG comprises or consists of the amino acid sequence VLQGVLPALPQVVCNYR.

The standard peptide for Inhibin-A may comprise an amino acid sequence selected from ALFLDALGPPAVTR, EGGDPGVR, HALGGFTHR, GLAQEAEEGLFR, YMFRPSQHTR, QVTSAQLWFHTGLDR, CPLCTCSARPEATPFLVAHTR, TRPPSGGER, STPLMSWPWSPSALR, LLQRPPEEPAAHANCHR, VALNISFQELGWER, TTSDGGYSFK and YETVPNLLTQHCACI. In a preferred embodiment the standard peptide for Inhibin-A comprises or consists of the amino acid sequence GLAQEAEEGLFR.

The standard peptide for PAPP-A may comprise an amino acid sequence selected from AGRPPRPAAGPATCATR, ASPPPPPPPGGAWEAVR, GATEEPSPPSR, DAFTLQVWLR, SPAVITGLYDK, GWVVGIHTISDQDNK, QVTTINAHR, SYLPGQWVYLAATYDGQFMK, LYVNGAQVATSGEQVGGIFSPLTQK, VLMLGGSALNHNYR, GYIEHFSLWK, VVNLYEDDHK, EQVDFQHHQLAEAFK, QYNISWELDVLEVSNSSLR, LILANCDISK, IGDENCDPECNHTLTGHDGGDCR, QHNGVCDMDCNYER, AYLDVNELK, LDGSTHLNIFFAK, SSEEELAGVATWPWDK, MHCYLDLVYQGWQPSR, ELGSACHLCLEGR, ILVQYASNASSPMPCSPSGHWSPR, EAEGHPDVEQPCK, NISLGPQNVFCDVPLTIR, DPPLQMDVASILHLNR, DQGEQCDDMNK, INGDGCSLFCR, QEVSFNCIDEPSR, CYFHDGDGVCEEFEQK, QETISVQLLDTK, DQSHDLGLHVLSCR, NNPLIIPVVHDLSQPFYHSQAVR, VSFSSPLVAISGVALR, SFDNFDPVTLSSCQR, GETYSPAEQSCVHFACEK, TDCPELAVENASLNCSSSDR, YHGAQCTVSCR, TGYVLQIR, SQTGPSVTVTCTEGK, CKPGYHVPGSSR, TQCTQDGSWQEGACVPVTCDPPPPK, FHGLYQCTNGFQFNSECR, CEDSDASQGLGSNVIHCR, DIPHWLNPTR, VVCTAGLK, WYPHPALIHCVK, GCEPFMGDNYCDAINNR, AFCNYDGGDCCTSTVK, VTPFPMSCDLQGDCACR and DPQAQEHSR. In a preferred embodiment the standard peptide for PAPP-A comprises or consists of the amino acid sequence CYFHDGDGVCEEFEQK.

The standard peptide for SAP may comprise an amino acid sequence selected from ESVTDHVNLITPLEKPLQNFTLCFR, AYSLFSYNTQGR, DNELLVYK, VGEYSLYIGR, QGYFVEAQPK, IVLGQEQDSYGGK and GYVIIKPLVWV. In a preferred embodiment the standard peptide for SAP comprises or consists of the amino acid sequence VGEYSLYIGR.

### Kits

The disclosure provides test kits for use in the methods of the invention. Such kits typically comprise an agent for the determination of the amount of a biomarker for DS. Such kits typically comprise instructions for carrying out a suitable method for diagnosing Down's syndrome in a human fetus comprising determining the amount of at least one biomarker for DS in a sample from the pregnant female carrying the fetus.

Any agent for the detection of a biomarker of DS may be used. Suitable agents are described herein. The agent for the detection of a biomarker of DS is preferably an antibody or fragment thereof, as described herein.

The agent for the determination of the amount of a biomarker of DS preferably comprises a sample comprising a known amount of at least one deuterated standard peptide derived from the a biomarker of DS. The deuterated standard peptide may be a deuterated form of any of the standard peptides disclosed herein for any DS biomarker. In a preferred embodiment of the disclosure, the biomarker of DS is C1 inhibitor and the kit comprises a deuterated standard peptide for C1 inhibitor. In a more preferred embodiment of the disclosure, the biomarker of DS is C1 inhibitor and the kit comprises a deuterated standard peptide for C1 inhibitor comprising the amino acid sequence FQPTLLTLPR.

The disclosure provides a test kit for use in a method for diagnosing DS by triple quadrupole mass spectrometry according to the present invention, said test kit comprising at least one deuterated standard peptide derived from at least one biomarker for DS. The test kit of the disclosure may be for use in diagnosing DS by MRM peptide analysis by triple quadrupole mass spectrometry.

The amount of C1 inhibitor may be measured alongside the level or concentration of other biomarkers for DS, such as SAP, complement C3, transthyretin, PAPP-A, free β-hCG, AFP, uE3, Inhibin-A and NT. At least one agent for the detection of one or more additional biomarkers for DS or agents for determining the amount of one or more additional biomarkers for DS may be included in the kit. The agent for the detection of the one or more additional biomarkers is preferably an antibody or fragment thereof, as described herein. The agent for the determination of the amount of the one or more additional biomarkers preferably comprises a sample comprising a known amount of at least one deuterated standard peptide derived from the one or more additional biomarkers.

In one embodiment of the disclosure, the test kit comprises at least one deuterated standard peptide for C1 inhibitor and at least one deuterated standard peptide from one or more additional biomarkers for DS. In a preferred embodiment, the one or more additional biomarkers for DS are selected from SAP, complement C3, transthyretin, β-hCG, PAPP-a, AFP, uE3 and Inhibin-A.

The kits of the disclosure may additionally comprise means for the measurement of other laboratory or clinical parameters.

The kits of the disclosure may additionally comprise one or more other reagents or instruments which enable any of the embodiments of the methods of the invention to be carried out. Such reagents or instruments include one or more of the following: suitable buffer(s) (aqueous solutions), means to isolate the at least one biomarker for DS from a sample, means to obtain a sample from the individual (such as a vessel, an instrument comprising a needle or a blood sample of Guthrie card) or a support on which quantitative reactions can be done.

The kits of the disclosure may, optionally, comprise instructions to enable the kit to be used in the method of the invention or details regarding which individuals the method may be carried out upon.

In one embodiment of the disclosure, additional diagnostic tests may be carried out to confirm the risk of DS. These additional tests may be carried out at the same time, before or after the method of the invention is carried out. Examples of such additional diagnostic tests that may be used are ultrasound, the cffDNA test, CVS and amniocentesis.

### Examples

### Example 1 - Identification of markers for DS

### Summary

Using ProteinChip Technology (SELDI TOF MS), the maternal plasma of 53 chromosomally normal control and 28 Down's syndrome affected pregnancies was profiled between 10 and 20 weeks' gestation. Preliminary studies demonstrated two distinct phases of changes in protein expression, the first at 10-14 weeks and second at 14-20 weeks.

Using this data, analysis of the 10-14 weeks' plasma samples (Down's syndrome n=13, control n=20) showed the presence of a protein of mass 100.3 kDa that was elevated in the Down's syndrome group compared to the controls (p<0.002). This protein was further isolated using SAX Q-spin columns and identified using QTOF MS and Western blotting as being plasma protease C1-inhibitor.

Analysis of the 14-20 week cohort demonstrated changes in protein expression of three additional proteins. Two of these proteins were found to be up-regulated (serum amyloid P-component, p<0.004 and transthyretin, p<0.006) and complement C3-α chain was observed to be down-regulated (p<0.0005).

### Methods

Plasma Samples: Blood (EDTA) were taken with consent from pregnant women attending the Fetal Medicine unit University College Hospital London for invasive diagnostic testing for a range of clinical indications as part of the SAFE Framework 6 Network of Excellence Study (Chitty et al (2008) Prenat. Diagn. 28: 83-88). Fifty three plasma samples were obtained from normal pregnancies and 28 from DS affected pregnancies. Plasma was obtained by centrifuging at 1500g for 10min and further separated by centrifuging at 16,000g for 15 min. protease inhibitor cocktail (Sigma Aldrich, UK) was added to 1ml aliquoted plasma fractions and stored at -80°C prior to analysis. The study was approved by the UCLHA Research Ethics committee (001/95).

### SELDI TOF Analysis

Whole plasma samples were thawed on ice. Samples were denatured by adding 10 µl of plasma to 90 µl 9M Urea 2%CHAPS (Sigma Aldrich UK) and shaken at room temperature for 30 min. Biorad Q10 anion exchange chips were used after preliminary studies using other protein arrays (CM10 and IMAC30) did not identify any potential biomarkers. Chips were secured in a bioprocessor and spot surfaces equilibrated with 100mM Tris-HCl pH 9 and 10 µl of the solubilised plasma/urea solution was added to 90 µl of binding buffer (100mM Tris-HCl pH9), samples were incubated for 1 hr on the chip surface with shaking. After incubation array surfaces were washed 3 times with binding buffer to remove any non-specifically bound proteins and a final wash in 5mM ammonium acetate pH 9 to remove salt residues. Spots were air dried and 2x 1 µl of Sinnapinic acid (Sigma Aldrich, UK) in 50% ACN, 0.05% TFA were added to each spot and air dried before being analysed by a SELDI TOF PBSIIc instrument.

Samples were analysed blind and arrays with the same batch number were used throughout the study. Two mass spectral analyses were performed on each array. The first involved a lower mass scan optimised to analyse proteins of mass 1.5-30 KDa. A second scan was performed to analyse proteins of higher molecular weight at up to 180 KDa with settings optimised between 50-110 KDa. After baseline subtraction spectra were normalised by means of the total ion current method. External calibration was performed using protein standards. Peak detection and statistical analysis were done by Biomarker Wizard software (Ciphergen) using a signal to noise ratio of three. All spectra were normalised from 1.5 kDa for the low mass scans and from 10 kDa for high mass scans.

Significant differential expression was determined by Students t-test with a value of <0.05 considered significant.

### Purification, isolation and Identification of proteins after detection by SELDI TOF MS

ProteinChip® Q spin columns (Biorad Laborotories) were used to isolate and fractionate plasma samples in sufficient amounts for identification. Columns were equilibrated briefly with binding buffer (9M Urea, 100mM Tris-HCL pH 9). Whole plasma was diluted 1:10 in binding buffer and incubated on the column for 40-60 min. The column was washed 3 times with 100mM ammonium bicarbonate buffer pH 9. Elution buffer consisted of 100mM ammonium bicarbonate buffer pH 2. Eluted protein (100µg) was analysed by SELDI on a blank gold chip and checked against normal maternal plasma Q10 chip spectra for any additional or missing peaks. This was used to confirm that the protein eluted from the ProteinChip® Q spin columns represented the same protein peaks observed on the Q10 chip.

The method was modified to further fractionate proteins by eluting them off on a pH gradient from 5.5 to 2. Eluted protein fractions were checked on a blank gold chip in the SELDI TOF MS.

Protein concentration was determined using the Bicinchoninic acid kit (Sigma). Eluted protein was then freeze-dried.

### SDSPAGE

Sample loads of 100 µg protein from aliquots purified using the preparative Q spin columns were heated at 90°c for 5 min in Laemmli loading buffer and resolved on 20% acrylamide SDS PAGE for smaller molecular weight proteins or 7.5% for the large molecular weight proteins. Gels were fixed and stained using 40% Methanol, 10% acetic acid, 0.25% Coomassie brilliant blue R-250.

### In-gel proteolytic digestion and extraction of peptides

In-gel digestions of proteins were performed as described by Shevchecko et al (Anal. Chem. (1996) 68: 850-858). Proteins were identified and quantitated by direct analysis of the reaction mixture described above. All analyses were performed using a nanoAcquity HPLC and QTOF Premier mass spectrometer (Waters Corporation, Manchester, UK). Peptides were trapped and desalted prior to reverse phase separation using a Symmetry C18 5µm, 5mm X 300µm pre-column. Peptides were then separated prior to mass spectral analysis using a 15cm X 75 µm C18 reverse phase analytical column. Peptides were loaded onto the pre-column at a flow rate of 4µl / min in 0.1% formic acid for a total time of 4 mins. Peptides were eluted off the pre-column and separated on the analytical column using a gradient of 3-40% acetonitrile [0.1% formic acid] over a period of 90 min and at a flow rate of 300 nl/min. The column was washed and re-generated at 300 nl/min for 10 min using a 99% acetonitrile [0.1%] rinse. After all non-polar and non peptide material was removed, the column was re-equilibrated at the initial starting conditions for 20 min. All column temp were maintained at 35°C. Mass accuracy was maintained during the run using a lock spray of the peptide [glu1]-fibrinopeptide B delivered through the auxiliary pump of the nanoAcquity at a concentration of 300 fmol/l and at a flow rate of 300 nl/min.

Peptides were analysed in positive ion mode using a Q-Tof Premier mass spectrometer (Waters Corp., Manchester, UK) and was operated in v-mode with a typical resolving power of 10,000 FWHM. Prior to analyses, the TOF analyser was calibrated using [glul]-fibrinopeptide B fragments obtained using a collision energy of 25 eV and over the mass range 50-2000 m/z. Post calibration of data files were corrected using the doubly charged precursor ion of [glu1]-fibrinopeptide B (785.8426 m/z) with a sampling frequency of 30 sec. Accurate mass LC-MS data were collected in a data independent and alternating, low and high collision energy mode. Each low/high acquisition was 1.5s with 0.1 sec interscan delay. Low energy data collections were performed at a constant collision energy of 4 eV, high collision energy acquisitions were performed using a 15-40eV ramp over a 1.5 sec time period and a complete low/high energy acquisition achieved every 3.2 sec. Samples were analysed using a Waters Premier QTOF and spectra processed using ProteinLynx Global Server version 2.3 (Waters Corporation, Manchester, UK) with a downloaded Uniprot Human Proteome database. Search settings allowed a minimum three ion matches per peptide, seven ion matches and three peptide matches per protein, 3 trypsin missed cleavages, with the carboxymethylated cysteine as fixed modification and oxidation of methionine as potential variable modification. Only identities from PLGS with 3 or more peptides were considered.

### Western Blot analysis

Dilutions of plasma samples were made in PBS and the equivalent of 0.75 µl of plasma were separated by either 10 or 15% SDS PAGE under reducing conditions. A standard control sample was selected and run on every blot to use as a calibration. Proteins were blotted onto Hybond-LFP PVDF membrane (GE Healthcare, Amersham, UK). Blots were blocked for 1 hr in 5% non fat dried milk in PBS. Membranes were incubated for 2 hrs at room temperature with either mouse 1 µg/ml monoclonal antibody to complement C3, 1:1000 dilution or mouse monoclonal antibody to serum amyloid P (SAMP) or 1:100 dilution of sheep polyclonal antibody to plasma C1-protease inhibitor (C1 inhibitor) that was labelled with Cy2 using a CyDye antibody labelling kit (GE Healthcare, Amersham, UK). All antibodies were optimised for dilution and purchased from Abcam Plc. Blots were washed 6 x10min with 0.1% Tween 20 in PBS before being incubated for 1 hr with anti mouse Cy3 labelled secondary antibody 1:2500 dilution (except in the case of C1 Inch). Blots were washed and dried overnight in the dark then scanned for the relevant Cy dye fluorescence using a Typhoon variable image scanner (Amersham Biosciences, Upsala, Sweden). ImageQuant software was used to measure volume, background intensity was subtracted, every blot was run in duplicate and intensity was calibrated and averaged for each sample. Statistical analysis was performed using Graphpad Prism 4 and a two tailed Students t-test was used to test for significance.

### Results

### Preliminary screens of maternal plasma samples using ProteinChip Technology (SELDI TOF MS).

A smaller study to the main analyses was performed using 16 maternal plasma samples (8 controls, 8 DS) and conducted using all 4 ProteinChip arrays, SAX (Q10), SCX (CM10), hydrophobic (H50) and immobilised-metal ion (IMAC). No significant differences in protein expression of maternal plasma proteins were observed using the SCX, hydrophobic or immobilised metal ion arrays. In contrast, several potential changes on protein expression were observed using the SAX array and it was decided therefore to perform a larger scale study using the SAX arrays (83 plasma samples).

### Gestational age timeline comparison

Initially, all data were not analysed by grouping into conventional gestational age categories (first and second trimesters of pregnancy) but analysed in 2 week gestational-age time points, in an attempt to accurately determine changes in the maternal plasma proteome and increase the potential for determining the specificity of potential biomarkers. Figure 1 shows overall changes in the maternal plasma proteome and how gestational age groups were defined into three distinct phases of change in protein expression. All peaks/proteins detected by the biomarker wizard software (Ciphergen) were analysed for significant differential expression of total peak volume in 2 week consecutive gestational age periods. The earliest period was 10-12 weeks which was compared with the next period of 12-14 weeks, where minimal change in the proteome was observed between these 2 periods and therefore these were grouped into a single 10-14 week group. This was then compared with next period of 14-17 weeks where a series of subtle but significant changes between these time periods were detected (this is unsurprising as this is when the second trimester commences). To reflect this, the degree of change is shown in Figure 1 as a percentage of 'significantly changed peaks/proteins' (p<0.05) of the total detected proteins in both groups. No further changes were observed between 14-17 weeks and 17-20 weeks, hence this time period was grouped into the 14-20 week group. A few samples beyond 20 weeks were compared with the 14-20 weeks group where some changes were seen as shown in Figure 1. This information was then used to define the final designated gestational age groups 10 -14, 14- 20 and 20 + weeks. When the corresponding DS gestational age groups were compared in this same way (DS 10-14 weeks vs DS 14-20 weeks etc) a delay, or lag, in the changes in proteomic expression in the DS group in comparison with controls was observed.

### Analysis of the 10-14 weeks gestational age plasma samples (SAX/Q10 array)

Figure 2 shows the results of the SELDI TOF MS analysis of maternal plasma from 20 control and 13 DS affected pregnancies. Only one protein of m/z 100.3 kDa, was detected as being statistically and significantly elevated in the DS group relative to the control group (p<0.00026). The mean concentration of this plasma protein was observed to be >1.5 fold higher in the DS affected pregnancies when compared to the control group (Table 1).

### Identification of the protein biomarker of m/z 100.3 kDa

Using ProteinChip Q spin columns (BioRad, UK), containing the same stationary phase as coated on the Q10/SAX array used to detect the biomarker, 5 x 20 µl of plasma was pooled from 5 DS affected maternal plasma and the biomarker isolated using a pH fractioning system. Protein fractions were eluted from the column using a series of decreasing pH washes. Each pH fraction was then freeze dried before analysis in the SELDI TOF MS to deduce the fraction that was most highly enriched with the potential biomarker of m/z 100.3 kDa (Figure 3). The pH 4 fraction was deduced to be the most highly enriched fraction for the potential biomarker (Figure 3) and was subsequently used in the identification analyses. The pH 4 fraction was freeze-dried prior to a further separation by ID SDS-PAGE, visualised using Coomassie Blue RG250 and the unknown protein of mass 100.3 kDa excised from the gel after comparison with molecular weight markers and the masses of other identified proteins in the sample. The gel piece was then subjected to in-gel tryptic digestion, peptides extracted in accordance with Mills et al 2001 and analysed by ESI-QTOF MS. Analysis by MS-based sequencing identified the protein as being plasma C1 protease inhibitor (Table 1). The two other major proteins detected in the pH 4 fraction of masses 66.9 kDa and 134.1 kDa were identified as being albumin and ceruloplasmin, respectively (Figure 3).

### Analysis of the 14-20 weeks gestational-age plasma samples (SAX/Q10 array)

SELDI TOF MS analysis of maternal plasma samples, of 33 controls and 15 DS affected pregnancies, identified 4 proteins that showed statistically significant changes in protein expression or post-translational modification (Table 1). A protein of m/z 23.4 kDa was shown to be elevated significantly (p<0.004) with on average an almost 3-fold elevation in the DS affected plasma samples (Figure 5) relative to the corresponding control group. In contrast, the expression of the protein of mass 112.5 kDa was statistically and significantly reduced (p<0.0005) in the Down's group compared the control cohort (Table 1). The protein of mass approx 14 kDa (13.88-14.14 kDa) demonstrated what appeared to be changes in post-translational modification with both gestational age and affected pregnancies (Figure 4).

### Identification of the plasma protein biomarkers in the 14-20 week gestational-age group

Similarly, to the identification of the 10-14 week biomarker described above, protein biomarkers identified by SELDI TOF MS were isolated and enriched using ProteinChip Q-spin columns and pH fractionation. Individual proteins were identified using molecular weight markers, excised, in-gel tryptically digested and the peptides eluted and sequenced using ESI-QTOF MS as described in Bennett et al (J. Proteome Res 9: 4289-4294, 2010). Tables 1 and 2 show the identification of the three potential biomarkers at m/z 14 kDa, 23.4 kDa and 112.5 kDa, as being transthyretin, serum amyloid P and complement C3, respectively. The use of SDS-PAGE, did not provide sufficient resolution to isolate the individual isoforms of transthyretin (Figure 4.). However, analysis of the combined band (13.9 -14.1 kDa) demonstrated that transthyretin was the only protein present and hence each mass difference was probably due to changes in post-translational modification of the protein (see discussion).

### Confirmatory and complementary analyses of all protein biomarkers found in the 10-14 weeks and 14-20 weeks gestational-age group

To confirm the identity of each protein biomarker, antibody-based methodology was again used to quantitate plasma C1 inhibitor, SAMP and Complement C3.

No confirmatory analyses were performed for transthyretin as the changes were probably due to post-translational modification and it was not possible to distinguish each isoform using polyacrylamide gel electrophoresis.

Western blotting analyses of maternal plasma C1 inhibitor in 17 control and 14 DS affected pregnancies (10-14 weeks) are shown in Figure 6. In agreement with the initial SELDI TOF MS analyses (Figure 2), plasma C1 inhibitor was significantly elevated in the Down's affected pregnancies by an average 1.7 fold (p<0.0229).

Similarly, Western Blotting analyses of maternal plasma SAMP from 15 control and 16 DS affected pregnancies (14-20 weeks) were also observed to agree with the initial SELDI TOF MS analyses and were significantly elevated (p<0.0155, Figure 7). This was an average 1.5-fold increase in the mean levels of SAMP in DS affected pregnancy samples versus the equivalent control group. However, reanalysis of maternal plasma from 16 control and 15 Down's affected pregnancies at 10-14 weeks did not show any significant changes in the amounts between groups. This was in agreement with the SELDI TOF MS analyses which also did not show elevated levels of SAMP during this gestational age range (Figure 7).

Complement C3 was the fourth protein identified in this study as being a potential biomarker for DS affected pregnancies. Peptides identified were found to cover the residues 723-1582 which indicate this to be the complement C3 alpha chain. Using SELDI TOF MS it was shown to be approximately 2-fold reduced in the 14-20 weeks Down's cohort of samples (p<0.00051, Table 1).

**Table 1. Proteins matched to differentially expressed masses on the SELDI TOF. Proteins were identified after purification using Q-Spin columns by MALDI/ESI QTOF MS/MS. Transthyretin was identified in three mass isoforms due to post-translational modification (unmodified 13.88 kDa, cysteinylated 14.05 kDa and gluthionylated 14.14 kDa).**

| **Gestation Period** | **SELDI Peak MW (kDa)** | **Accession no.** | **Entry & Description** | **Fold change in DS** | **p Value** |
|---|---|---|---|---|---|
| First Trimester (10-14wks) | 100.3 | P05155 | IC1_HUMAN Plasma C1 protease Inhibitor precursor | ↑ 1.53 | 0.00026 |
| Second Trimester (14-20wks) | 23.4 | P02743 | SAMP_HUMAN Serum Amyloid P precursor | ↑ 2.8 | 0.004 |
| | 112.5 | P01024 | CO3_HUMAN Complement C3 precursor | ↓ 2.01 | 0.00051 |
| | 13.88 | P02766 | TTHY_HUMAN Transthyretin | ↑ 1.54 | 0.006 |
| | 14.0 | | | ↓ 1.3 | 0.022 |
| | 14.14 | | | ↓ 1.3 | 0.005 |

**Table 2. Identification of the proteins separated by SDS PAGE. Peptide analysis was performed by ESI-QTOF and data analysed using Protein Lynx global server v.2.3. Proteins with a protein PLGS score higher than 9 were considered as a 100% confident identification.**

| **SELDI peak MW (kDa)** | **Accession No.** | **Protein entry and description** | **Theoretical MW (kDa)** | **Theoretical PI** | **PLGS score** | **% coverage** | **No. of identified peptides** |
|---|---|---|---|---|---|---|---|
| 13.88-14.14 | P02766 | TTHY_HUMAN Transthyretin | 15.9 | 5.4 | 11.6 | 63.94 | 6 |
| 23.5 | P02743 | SAMP_HUMAN Serum Amyloid P component | 25.37 | 6.1 | 11.6 | 15.7 | 3 |
| 100.3 | P05155 | IC1_HUMAN Plasma C1 protease Inhibitor precursor | 55.12 | 6.1 | 11.6 | 22 | 12 |
| 112.5 | P01024 | CO3_HUMAN Complement C3 precursor | 187.05 | 5.55 | 11.6 | 11.54 | 15 |

### Example 2 - Triple Quadrupole Mass Spectrometry

### Summary

A single-reaction-monitoring (SRM) tandem mass spectrometry-based assay was created for the quantitation of the biomarkers identified in Example 1. These results were compared with an in-house developed immunofluorescence-based technique (IF). The new MS-based assay is a rapid 5 min test and a simple "one pot reaction," requiring only 5 µl of plasma.

To evaluate the potential of SRM-based quantitation in a clinical setting, SAMP and C1-inhibitor were quantitated in 38 normal and Down syndrome affected pregnancies. Plasma SAP levels in the Down's group were significantly raised at 10-14 weeks (p<0.0015) and 14-20 weeks (p<0.0001). Plasma C1-inhibitor levels were also observed significantly elevated in the Down's group (10-14 weeks, p<0.0193, 14-20 weeks, p<0.0001). Analysis using the IF technique did not show any significant elevation of plasma SAP levels or C1-inhibitor levels.

This rapid and sensitive assay demonstrates the potential of multiplexed tandem MS-based quantitation of proteins in chemical pathology labs and in a more cost-effective, accurate manner than conventionally used antibody methods.

### Methods

### Plasma samples

Blood samples (EDTA) were taken with ethical consent from 38 pregnant women attending the Fetal Medicine Unit, University College Hospital London for invasive diagnostic testing for a range of clinical indications. Nineteen plasma samples were obtained from normal pregnancies and nineteen from DS affected pregnancies. Plasma was obtained by centrifuging at 1,500g for 10 min and further separated by centrifuging at 16,000g for 15 min. Protease inhibitor cocktail (Sigma Aldrich, UK) was then added to 1 ml aliquoted plasma fractions and fractions were stored at -80 °C prior to analysis. The study was approved by the UCLH A Research Ethics committee (001/95).

### Sample preparation: The in-solution digestion of plasma proteins prior to analysis by UPLC MS/MS

A 5 µl sample of whole plasma and 50 pmol of peptide AQUA internal standard (5 µl of 10 nmol/ml 50 mM ammonium bicarbonate pH 8.2 solution) was added to 20 µl of 100mM Tris, pH 7.8 containing 6M urea and was left to shake at room temperature for 1 hr. Disulphide bridges were reduced by the addition of 3 µl of 100 mM Tris-HCL, pH 7.8 containing 5M DTE and incubation at room temperature for 60 min. Free thiol groups were carboamidomethylated followed by incubation with 6 µl of 100 mM Tris-HCL, pH 7.8 containing 5M iodoacetamide. The solution was then diluted with 150 µl H₂O, vortexed and 2 µg of sequence grade trypsin (Sigma, Aldrich UK) added to the solution. Samples were incubated overnight at 37 °C in a water bath. Digests were spun at 12,000g for 10 min and the supernatant was analysed by LC MS.

### Development of triple quadrupole method for absolute quantitation of C1 inhibitor and SAMP in tryptically digested plasma.

Figure 11 shows a schematic representation summarising the assay used in the quantitation of C1 inhibitor/SAMP.

Data obtained for MALDI-TOF identification was used to select two candidate peptides for protein quantitation by tandem LC/MS. Peptides *m*/*z* 913.98 GVTSVSQIFHSPDLAIR and *m*/*z* 593.35 FQPTLLTLPR from C1 inhibitor were selected and peptides *m*/*z* 703.84 AYSLFSYNTQGR and *m*/*z* 579.79 VGEYSLYIGR from SAMP were selected for custom synthesis (Genscript Corp, NJ, USA).

ESI-QToF analyses of tryptic peptides derived from C1-inhibitor and SAP protein standards showed that the peptides m/z 593.54 (FQPTLLTLPR) from C1-inhibitor and m/z 578.79 (VGEYSLYIGR) from SAP produced both the largest response and optimum product ion scans for C1-inhibitor and SAP analyses, respectively. These peptides were thus chosen as the "marker" peptides for each protein and peptide standards were obtained commercially to be used for optimization and development of the SRM-based assay (GenScript Corp., NJ, USA). An internal standard for the SAP peptide was also synthesised, which included a single missed cleavage site with a short peptide "tag" (VGEYSLIGR[ 13C2 15N]-HKVTS) to compensate for any inefficiencies of the trypsin protease reaction ("Aqua™ peptide," Sigma-Aldrich, Dorset, UK). Confirmation of the identification of the marker peptides for SAP m/z 578.79 and C1-inhibitor m/z 593.54 in plasma was verified by comparison of their retention time with the synthesised versions of the peptides when spiked into blood. In this way a third level of identification was attained in addition to precursor and product ion mass and enabling unequivocal identification of each peptide/protein. Quantitative data were taken from transition 593.54>455.980 for C1 inhibitor, 578.79>286.199 for SAP and 580.31>286.199 for the deuterated internal standard. Calibration curves were constructed by spiking 5 µl of normal female plasma with 50 pmol of the SAP isotope labelled internal standard and increasing amounts of the synthesised marker peptides used to quantitate the SAP and C1-inhibitor proteins (1, 2.5, 5, 7.5, 10 and 15 µmol/l).

Endogenous levels of SAP and C1-inhibitor levels in the normal female plasma were determined by the intercept on the x-axis of the unspiked plasma (0 calibrator point) and this correction value was subtracted from each subsequent calibrator point (1-15 µmol/l). Quantitation of SAP and C1-inhibitor was performed by ratioing the peak area of the native peptide to the peak area of isotope-labelled internal standard for SAP (580.31 m/z) and the concentration determined by comparison to the calibration curve. Results were expressed as (µmol/l.

A Waters 2795 HPLC coupled to a Quattro Micro ^{TM} triple quadrupole mass spectrometer was used. The instrument was operated in positive ion mode; pure peptides were directly infused into the electrospray source via a 25 mm (i.d.) fused silica transfer line by means of a Harvard syringe pump at a flow rate of 25 µl/min. The capillary voltage was maintained at 3.7 kV with a cone voltage of 24 V. The source temperature was held constant at 150 °C and nitrogen was used as the nebulising gas at a flow rate of 30 l/h. The masses were determined for each peptide in scan mode in a mass range of *m*/*z* 400 -1300. Product ions were determined over a mass range of *m*/*z* 50-1200 following collision-induced dissociation and using argon as the collision gas. Optimum cone and collision energies for each peptide are listed in Table 2.

Only peptides 593.35 from C1 inhibitor and 578.79 from SAMP produced useable product ion data and for quantitation 2 transitions from each peptide were selected for confirmation purposes. An HPLC profile was created and optimised to these transitions using an HS F5 5 cm x 2.1 mm, 5 um (Supelco USA). The peptide elution gradient consisted of a total of 29 min starting from 3 to 40 % ACN over 20 min, then to 100 % ACN over 5 min and back to 3 % ACN over the remaining 4 min at a flow rate of 0.25 mL/min and a column temp of 30 °C. Two transitions were used for each targeted tryptic peptide, with dwell times set at 0.05 s, and relevant collision energies (Table 2). MRM transitions for SAMP and C1 inhibitor are also listed in Table 2.

**Table 3 shows MRM transitions for the proteins used in the LC-MS analysis. All precursor ions are doubly charged.**

| **Protein** | **Tryptic peptide sequence** | **Precursor m/z** | **Product ion m/z** | **Product ion m/z** | **Cone voltage** | **Collision energy** |
|---|---|---|---|---|---|---|
| Plasma C1 inhibitor | FQPTLLTLPR | 593.28 | 455.98 | 276.3 | 23 | 20.0 |
| Serum amyloid P | VGEYSLYIGR | 579.799 | 286.03 | 508.54 | 25 | 19.0 |
| AQUA® Peptide | VGEYSL^{[C13N15]}YIGR | 581.43 | 286.03 | 508.54 | 25 | 19.0 |

### Immunofluorescent Western blot analysis

An immunofluorescence based assay was optimised and developed to detect both SAP, C1-inhibitor. The optimum volume of plasma to be used was found to be 0.75 µl (data not shown) and proteins were separated under denaturing conditions on a 10% [0.1% bis-acrylamide] SDS 1D-PAGE.

Proteins were blotted onto Hybond-LFP PVDF membrane (GE Healthcare, Amersham, UK). Blots were blocked for 1 h in 5% (w/v) non-fat dried milk in PBS. Membranes were incubated for 2 h at room temperature with either mouse monoclonal antibody to SAP (1:1000 dilution) or 1:100 dilution of sheep polyclonal antibody to C1-inhibitor that was labelled with fluorescent cy dye Cy2 using a CyDye™ antibody labelling kit (GE Healthcare, Amersham, UK). All antibodies were purchased from Abcam and optimised for dilution. Blots were washed 6×10 min with 0.1% (v/v) Tween 20 in PBS before being incubated for 1 h with anti-mouse fluorescent cy dye Cy3 labelled secondary antibody 1:2500 dilution (except in the case of C1 inhibitor). Blots were washed and dried overnight in the dark then scanned for the relevant Cy dye fluorescence using a Typhoon Trio variable image scanner (GE Healthcare, Amersham, UK). ImageQuant TL software (GE Healthcare, Amersham, UK) was used to measure volume and background intensity was subtracted. Immunofluorescence readings for C1-inhibitor and SAP were standardised against anti-IgG levels (Santa Cruz Biotechnology CA). Every blot was run in duplicate and intensity was calibrated and averaged for each sample. Statistical analysis was performed using GraphPad Prism 4 and a two tailed Students t-test p value <0.05 was considered significant.

A custom AQUA peptide (Sigma UK) was synthesised based on the SAMP 579.79 peptide to use as an internal standard for absolute quantitation of SAMP and which was also used in the quantitation for C1 inhibitor in plasma. A subsequent missed trypsin site and amino acid tag was incorporated in order to control for variable trypsin digestion (VGEYS[L^{C13N15]}YIGRHKVTS).

### Results

Native SAMP 579.79 and C1 inhibitor 593.35 in digested plasma were identified by comparison of retention time with the synthesised versions of the peptides (Figure 8).

Preliminary experiments were performed to optimise the amount of plasma to assay wand the amount of SAMP peptide internal standard to use. Linear quantitation of SAMP 579.79 and C1 inhibitor 593.35 in trypsin digested whole plasma was not observed until 5 ul, an increase of cone voltage was observed with plasma digests more than 10 µl Therefore, the optimum amount of plasma to assay was determined to be 5 µl. A standard curve of increasing amounts of custom made SAMP peptide 579.79 in 5 µl of whole plasma with 50 pmols AQUA SAMP peptide was created to confirm a linear relationship. Endogenous SAMP was assayed and subtracted from the values (Figure 9).

Data was analysed using Masslynx version 4.0. Integrated peak values for endogenous SAMP *m*/*z* 579.79 and internal standard SAMP AQUA peptide *m*/*z* 581.79 were used to obtain values in µmol/L endogenous SAMP. Ten plasma samples for each group were analysed in duplicate using Microsoft Excel. Students T-test was used to determine significance.

This investigation was optimised further to produce a more accurate and reproducible LC/MS peptide based assay for C1 inhibitor and SAMP. Ten samples for each group were assayed. The assay was set up to monitor 2 transitions per peptide.

The most specific transition without contaminating peaks were selected for quantitation and were 579.79>508.54 for SAMP and 593>455.98 for C1 inhibitor. The HPLC profile used for separation was based on the same profile used by the QTOF MS/MS that was used for identification. The tandem LC/MS SAMP results confirm the SELDI and western blot experiments and show a clear increase in both gestational age periods (Figure 10A). Previously SAMP could not be detected in the 10-14 week groups by SELDI and differential expression could not be proved in that group by western blotting (Figure 7). However the MRM peptide analysis by triple quadrupole mass spectrometry assay developed proved to be more sensitive and demonstrated significant 2-fold up-regulation in the 10-14 week DS group.

Much more variation was observed in comparison with the later gestational age group, which may be why a clear differentiation could not be demonstrated by the other methods. The 14-20 week group shows an almost complete differentiation in the DS group of 2.12-fold and corroborates the findings of the other methods. The significance (p<0.00015) compared with other methods (p<0.012 Western blot, p>0.004 SELDI), which indicates the triple quadrupole mass spectrometry assay was a much more accurate, sensitive and reproducible test.

The C1 inhibitor results (Figure 10B) also show significant increase in the DS groups at both gestational ages by a similar fold change seen by the western blot method (appx 1.7-fold).

The present inventors have shown in this study that serum proteins can be quantitated very accurately using a triple quadrupole mass spectrometry method using only 5 µl plasma. This method could be applied to the other existing markers for DS to create a single test. LC/MS is widely used to measure small molecules such as metabolites but has not been applied to measure proteins. However, when protein is digested to peptides then this method can be used to quantitate a specific peptide which can be related to that protein. This method has been successfully used to monitor naturally occurring peptides in serum (van den Broek et al (2010) J. Chromatogr. B Analyt. Technol. Biomed. Life Sci. 878 (5-6): 590-602) and also for tryptically digested plasma protein (Kay et al (2007) Rapid Commun. Mass Spectrom. 21(16): 2585-2593).

### Development of a triple quadrupole mass spectrometry-based assay for the quantitation of C1-inhibitor and SAP

Preliminary experiments were performed to optimise the amount of plasma for assay and the amount of SAP Aqua™ peptide internal standard to use. The limits of detection (signal to noise 3:1) of both SAP and C1-inhibitor peptides when spiked into plasma were found to be 0.1 µmol/l and were linear over the concentration range of 0.1-50 µmol/l (Fig. 12A and B). The ranges for SAP found in control and DS affected plasma samples were found to be 0.88-2.68 and 1.94- 6.32 µmol/l, respectively. The range for C1-inhibitor found in control and DS affected plasma samples was 0.48-2.85 and 1.38-5.93 µmol/l, respectively. All ranges were well within the linear range of the calibration curves used (Fig. 12A and B).

Further optimisation of the amount of plasma to be used was conducted by the digestion of increasing amounts of plasma to evaluate response, limits of detection and ion suppression. Observing the effect of the volume of plasma digested on the response of 50 pmols on the SAP internal standard demonstrated significant ion suppression at volumes greater than 5 µl of plasma (Fig. 12C). Therefore, a volume of 5 µl of plasma was determined as the optimum volume for analysis between ion suppression effect and the volume of plasma required for detection of the C1-inhibitor and SAP proteins. Calibration curves were constructed by spiking 5 µl of normal female plasma with increasing amounts of the marker peptides used to quantitate SAP and C1-inhibitor (1, 2.5, 5, 7.5, 10 and 15 µmol/l). Rapid chromatographic run times of 5 min were performed using UPLC chromatography with the standard eluting times at approximately 1.87 min for SAP and approximately 2.39 min for C1-inhibitor, respectively (Fig. 13). Intrabatch variation for plasma SAP and C1-inhibitor was deduced by the repeated analysis of a plasma sample from a Down's affected pregnancy (measured values of SAP and C1-inhibitor of 3.3 and 3.0 µmol/l, respectively). A total of 30 analyses of the same sample gave an intra-batch coefficient of variation (CV%) of 5.1% and 6.1% for SAP and C1-inhibitor protein, respectively. Inter-batch variation of plasma SAP was determined by the repeated analysis of the same sample (×10) over 3 separate weeks and was determined to be 7.4% and 8.5% for plasma SAP and C1-inhibitor protein, respectively.

### Quantitation of SAP and C1-inhibitor protein in maternal plasma in high risk normal and Down's affected pregnancies

Fig. 14 shows the maternal plasma levels of both SAP (Fig. 14a) and C1-inhibitor protein (Fig. 14b) in all Down's affected and high risk normal pregnancies. Plasma SAP levels were significantly elevated in the Down syndrome affected group, with a mean plasma SAP level of 3.46 µmol/l (range 1.94- 6.32 µmol/l) compared to 1.64 µmol/l (range 0.88-2.68 µmol/l) in the high risk normal group (p=0.0001). This equates to a greater than 2-fold elevation of the plasma SAP levels in the Down's group compared to the controls. Similarly, plasma levels of C1-inhibitor (Fig. 14b) were also significantly elevated in the Down syndrome affected group (p=0.0008). Mean plasma concentrations of C1-inhibitor in the Down's affected group were 2.30 µmol/l (range 1.38-5.93 µmol/l) relative to a level of 1.31 µmol/l (range 0.48-2.85 µmol/l) in the high risk normal group. This figure equates to an approximate 1.8-fold elevation in the mean plasma C1-inhibitor levels.

### Quantitation of SAP and C1-inhibitor protein in maternal plasma in high risk normal and Down's affected pregnancies according to trimester of pregnancy

The 38 patient samples were divided into first (10-14 weeks) and second (14-20 weeks) trimesters for analysis. Significant differences in the maternal plasma levels of both SAP and C1- inhibitor were observed between Down's affected and high risk normal pregnancies in both the first and second trimester analyses (Fig. 15A). Mean plasma SAP levels at 10-14 weeks in the normal and Down's groups were 1.78 µmol/l (range 1.19- 2.68 µmol/l) and 3.78 µmol/l (range 1.94-6.32 µmol/l), respectively.

This was a greater than 2-fold increase in the mean SAP levels in the Down's group versus the normal controls and had a statistical significance of p=0.0015. Mean plasma SAP levels in the second trimester were 3.41 µmol/l (range 2.55-4.07 µmol/l) and 1.53 µmol/l (range 0.88-2.74 µmol/l) in the Down's affected and normal group, respectively, and was an even greater statistical significance than was observed in the first trimester analyses with p<0.0001 (Fig. 15A).

Comparison of the mean plasma C1-inhibitor levels between the normal and DS affected pregnancies at both 10- 14 weeks and 14-20 weeks showed that the levels were statistically significantly different between the two groups, with p<0.0193 for the first trimester and increasing in significance to p<0.0001 in the second trimester (Fig. 15B).

The mean plasma C1-inhibitor levels in the first trimester DS affected and normal groups were 2.74 µmol/l (range 1.38-5.93 µmol/l) and 1.54 µmol/l (range 0.94-2.85 µmol/l), respectively (Fig. 15B), an approximate 1.8-fold increase in the DS group. Analysis of the 2nd trimester mean plasma C1-inhibitor levels showed 2.03 µmol/l (range 1.49-2.48 µmol/l) and 1.1 µmol/l (range 0.48-1.71 µmol/l) in the DS and normal groups, respectively. This was an approximate 1.8 fold increase in the DS affected group versus the normal controls.

### Maternal plasma SAP and C1-inhibitor protein levels analysed using immunofluorescence based methods

Relative levels of SAP and C1-inhibitor were compared between DS and control maternal plasma samples from 1^{st} trimester patients only (Table 4, Fig. 16). In contrast to the tandem MS analyses (Fig. 15), no significant differences were detected between the control and DS affected pregnancy groups for both SAP (p=0.15) and C1-inhibitor protein (p=0.24).

### The effects of gestational age, foetal sex and maternal weight on plasma SAP and C1-inhibitor plasma levels

Analysis of maternal plasma SAP and C1-inhibitor levels by gestational age of the foetus showed that both proteins increase in some patients in the DS group during the 1 st trimester of pregnancy, although no significant statistical difference was observed. Plasma levels of both SAP and C1-inhibitor in the control group did not demonstrate any significant change with gestational age of the foetus.

Comparison of foetal sex with maternal plasma SAP and C1-inhibitor levels in normal control pregnancies demonstrated a statistically significant difference of p=0.034. The mean SAP plasma concentration in the pregnancies carrying male foetuses was 1.91 µmol/l compared to 1.39 µmol/l in those pregnancies carrying female foetuses, an approximate 1.4-fold increase. Comparison of maternal plasma SAP concentrations when male and female foetuses were present in DS affected pregnancies demonstrated no significant statistical difference. No significant correlations between foetal sex and plasma C1-inhibitor protein concentrations were observed in either normal control or DS affected pregnancies.

Linear regression analyses showed no significant changes in plasma SAP concentrations over the maternal age-range 18- 47 years in both control and DS syndrome affected pregnancies. Similarly, no significant differences were observed in plasma C1-inhibitor levels in the control normal pregnancy group. However, linear regression analyses of maternal plasma C1- inhibitor levels in the DS affected pregnancies did show a statistically significant difference.

The final parameter evaluated was the potential effect of maternal weight on plasma SAP and C1-inhibitor levels. The relationship between SAP and C1-inhibitor with maternal weight was assessed in both normal control and DS affected pregnancies, respectively. Linear regression and grouped analyses of both categories demonstrated no significant association between maternal weight and plasma SAP and C1-inhibitor levels.

Using immunochemical methods we were not able to show any statistical significant difference between plasma SAP or C1-inhibitor levels between the DS and control pregnancy groups in the 1st trimester of pregnancy (p=0.15 and p=0.24, respectively). However, using SRM-based methods and the improved specificity of MS, it was possible to show there was a highly statistical significant difference in plasma SAP levels between the two groups during this time period (p<0.0015). This indicates that using MS-based protein quantitation over IF could significantly improve the specificity of the combined test currently used in prenatal screening of DS, thereby decreasing the false positive rate and reducing the number of unnecessary invasive tests with the concomitant miscarriage risk.

In addition, a total of 10-20 ml of whole blood is taken for the IF-based 1^{st} trimester biochemical screening test but our SRM-based assay only requires 5 µl of plasma. This ability to use smaller volumes of plasma to quantitate diagnostic markers in the circulation indicates that a blood spot or Guthrie card type blood collection may be used. This collection procedure would avoid the need for phlebotomy.

### Sequence Information

The amino acid sequence of human C1 inhibitor (UniProt Accession No. P05155) is shown below:

MASRLTLLTL LLLLLAGDRA SSNPNATSSS SQDPESLQDR GEGKVATTVI SKMLFVEPIL 60
EVSSLPTTNS TTNSATKITA NTTDEPTTQP TTEPTTQPTI QPTQPTTQLP TDSPTQPTTG 120
SFCPGPVTLC SDLESHSTEA VLGDALVDFS LKLYHAFSAM KKVETNMAFS PFSIASLLTQ 180
VLLGAGENTK TNLESILSYP KDFTCVHQAL KGFTTKGVTS VSQIFHSPDL AIRDTFVNAS 240
RTLYSSSPRV LSNNSDANLE LINTWVAKNT NNKISRLLDS LPSDTRLVLL NAIYLSAKWK 300
TTFDPKKTRM EPFHFKNSVI KVPMMNSKKY PVAHFIDQTL KAKVGQLQLS HNLSLVILVP 360
QNLKHRLEDM EQALSPSVFK AIMEKLEMSK FQPTLLTLPR IKVTTSQDML SIMEKLEFFD 420
FSYDLNLCGL TEDPDLQVSA MQHQTVLELT ETGVEAAAAS AISVARTLLV FEVQQPFLFV 480

**Table 4 - Comparison of MRM based assay with immunofluorescence based assay of maternal plasma concentrations of SAMP and C1-inhibitor protein in normal control and DS affected pregnancies**

| Group | Marker | SRM method mean ±SD (µmol/l) | p value | Immunofluroescence method (SAMP and C1 inhibitor/IgG) mean ±SEM (response) | p value |
|---|---|---|---|---|---|
| Control 10-14 weeks | SAMP | 1.78 ± 0.38 | 0.0015 | 0.0019 ± 0.000010 | 0.15 |
| DS 10-14 weeks | | 3.78 ± 1.60 | | 0.002 ± 0.00011 | |
| Control 10-14 weeks | C1 inhibitor | 1.54 ± 0.59 | 0.019 | 0.00105 ± 0.00016 | 0.24 |
| DS 10-14 weeks | | 2.74 ± 1.36 | | 0.0051 ± 0.00033 | |

## Claims

1. A method for diagnosing Down's syndrome (DS) in a human fetus comprising determining the amount of plasma protease C1 inhibitor (C1 inhibitor) in a sample from the pregnant female carrying the fetus.

2. The method of claim 1, further comprising comparing the amount of C1 inhibitor in the sample with the amount of C1 inhibitor in:
(a) a sample from a non-pregnant female; and/or
(b) a sample from a pregnant female carrying a fetus that does not have DS.

3. The method of any one of the preceding claims wherein the level of C1 inhibitor is determined by a method comprising:
(a) triple quadrupole mass spectrometry;
(b) mass spectrometry;
(c) an Enzyme-linked Immunosorbant Assay (ELISA); and/or
(d) immunofluorescence.

4. The method of any one of the preceding claims, wherein said method further comprises detecting or determining the amount of one or more additional biomarkers for DS.

5. The method of claim 4, wherein said one or more additional biomarker for DS is selected from complement C3, transthyretin, serum amyloid protein (SAP), the β subunit of human chorionic gonadotrophin (β-hCG), pregnancy-associated plasma protein A (PAPP-a), alphafetoprotein (AFP), unconjugated estriol (uE3) and Inhibin-A.

6. The method of claim 3, wherein the method comprises triple quadrupole mass spectrometry and the amount of C1 inhibitor is determined by comparing the amount of at least one marker peptide for C1 inhibitor within the sample with a known amount of at least one deuterated standard peptide derived from C1 inhibitor.

7. The method of claim 6, wherein said biomarker is C1 inhibitor and the at least one deuterated standard peptide comprises the amino acid sequence FQPTLLTLPR.

8. The method of any one of the preceding claims, wherein the pregnant female is in the first or second trimester of pregnancy.

9. The method of any one of the preceding claims wherein the sample is a sample of blood, serum or plasma from the pregnant female.

10. The method of claim 8, wherein the sample is collected on a Guthrie card.

11. The method of claim any one of the preceding claims, wherein the volume of the sample is less than 50 µl.

## Patentansprüche

1. Verfahren zum Diagnostizieren des Down-Syndroms (DS) in einem humanen Fötus, das das Bestimmen der Menge an Plasma-Protease-C1-Hemmer (C1-Hemmer) in einer Probe der schwangeren Frau, die den Fötus in sich trägt, umfasst.

2. Verfahren nach Anspruch 1, das des Weiteren das Vergleichen der Menge an C1-Hemmer in der Probe mit der Menge an C1-Hemmer in:
(a) einer Probe einer nicht schwangeren Frau; und/oder
(b) einer Probe einer schwangeren Frau, die einen Fötus in sich trägt, der kein DS hat, umfasst.

3. Verfahren nach einem der vorstehenden Ansprüche, wobei der C1-Hemmer-Spiegel durch ein Verfahren bestimmt wird, das umfasst:
(a) Triple-Quadrupol-Massenspektrometrie;
(b) Massenspektrometrie;
(c) einen Enzyme-linked Immunosorbant Assay (ELISA); und/oder
(d) Immunfluoreszenz.

4. Verfahren nach einem der vorstehenden Ansprüche, wobei das Verfahren des Weiteren das Nachweisen oder Bestimmen der Menge eines oder mehrerer zusätzlicher Biomarker für DS umfasst.

5. Verfahren nach Anspruch 4, wobei der eine oder die mehreren zusätzlichen Biomarker für DS aus Komplement C3, Transthyretin, Serum-Amyloid-Protein (SAP), der β-Untereinheit von humanem Choriongonadotrophin (β-hCG), schwangerschaftsassoziiertem Plasma-Protein A (PAPP-a), alpha-Fetoprotein (AFP), unkonjugiertem Östriol (uE3) und Inhibin-A ausgewählt ist bzw. sind.

6. Verfahren nach Anspruch 3, wobei das Verfahren Triple-Quadrupol-Massenspektrometrie umfasst und die Menge an C1-Hemmer durch Vergleichen der Menge zumindest eines Markerpeptids für C1-Hemmer in der Probe mit einer bekannten Menge zumindest eines deuterierten Standardpeptids, das von einem C1-Hemmer abgeleitet ist, bestimmt wird.

7. Verfahren nach Anspruch 6, wobei der Biomarker ein C1-Hemmer ist und das zumindest eine deuterierte Standardpeptid die Aminosäuresequenz FQPTLLTLPR umfasst.

8. Verfahren nach einem der vorstehenden Ansprüche, wobei die schwangere Frau im ersten oder zweiten Schwangerschaftstrisemester ist.

9. Verfahren nach einem der vorstehenden Ansprüche, wobei die Probe eine Blut-, Serum- oder Plasmaprobe der schwangeren Frau ist.

10. Verfahren nach Anspruch 8, wobei die Probe auf einer Guthrie-Karte gesammelt wird.

11. Verfahren nach einem der vorstehenden Ansprüche, wobei das Volumen der Probe weniger als 50 µl beträgt.

## Revendications

1. Procédé de diagnostic du syndrome de Down (SD) chez un foetus humain, comprenant la détermination de la quantité de C1-inhibiteur de protéase plasmatique (C1-inhibiteur) dans un échantillon prélevé sur la femme enceinte porteuse du foetus.

2. Procédé de la revendication 1, comprenant en outre la comparaison de la quantité de C1-inhibiteur dans l'échantillon à la quantité de C1-inhibiteur dans :
(a) un échantillon prélevé sur une femme non enceinte ; et/ou
(b) un échantillon prélevé sur une femme enceinte porteuse d'un foetus qui n'est pas atteint du SD.

3. Procédé de l'une quelconque des revendications précédentes, dans lequel le taux de C1-inhibiteur est déterminé par un procédé comprenant :
(a) une spectrométrie de masse de type triple quadripôle ;
(b) une spectrométrie de masse ;
(c) un essai d'immuno-absorption enzymatique (ELISA) ; et/ou
(d) une immunofluorescence.

4. Procédé de l'une quelconque des revendications précédentes, ledit procédé comprenant en outre la détection ou la détermination de la quantité d'au moins un biomarqueur supplémentaire pour le SD.

5. Procédé de la revendication 4, dans lequel ledit au moins un biomarqueur pour le SD est choisi parmi le complément C3, la transthyrétine, la protéine amyloïde sérique (SAP), la sous-unité β de la gonadotrophine chorionique humaine (β-hCG), la protéine A plasmatique associée à la grossesse (PAPP-a), l'alphafoetoprotéine (AFP), l'estriol non conjugué (uE3) et l'inhibine-A.

6. Procédé de la revendication 3, le procédé comprenant une spectrométrie de masse de type triple quadripôle et la quantité de C1-inhibiteur est déterminée par comparaison de la quantité d'au moins un marqueur peptidique pour le C1-inhibiteur dans l'échantillon à une quantité connue d'au moins un peptide classique deutéré dérivé du C1-inhibiteur.

7. Procédé de la revendication 6, dans lequel ledit biomarqueur est le C1-inhibiteur et l'au moins un peptide classique deutéré comprend la séquence d'acides aminés FQPTLLTLPR.

8. Procédé de l'une quelconque des revendications précédentes, dans lequel la femme enceinte en est au premier ou second trimestre de grossesse.

9. Procédé de l'une quelconque des revendications précédentes, dans lequel l'échantillon est un échantillon sanguin, sérique ou plasmatique de la femme enceinte.

10. Procédé de la revendication 8, dans lequel l'échantillon est recueilli sur une carte de Guthrie.

11. Procédé de l'une quelconque des revendications précédentes, dans lequel le volume de l'échantillon est inférieur à 50 µL.
